# FASCICULE DE BREVET EUROPEEN

(11) **EP 2 334 430 B1**
(45) Date de publication et mention de la délivrance du brevet: **26.02.2014**
(21) Numéro de dépôt: 09752879.8
(22) Date de dépôt: 24.09.2009
(51) Int. Cl.: B01J 37/00, B01J 29/03, B01J 29/04, B01J 35/00

(54) **PROCEDE DE PREPARATION D'UN MATERIAU POREUX STRUCTURE COMPORTANT DES NANOPARTICULES DE METAL 0 INCORPOREES DANS LES MURS**
VERFAHREN ZUR HERSTELLUNG EINES STRUKTURIERTEN PORÖSEN MATERIALS, UMFASSEND IN DIE WÄNDE DAVON EINGEBETTETE METALL-0-NANOPARTIKEL
METHOD FOR PREPARING A STRUCTURED POROUS MATERIAL COMPRISING NANOPARTICLES OF METAL 0 IMBEDDED IN THE WALLS THEREOF

(30) Priorité: 07.10.2008 FR 0856803
(43) Date de publication de la demande: 22.06.2011
(73) Titulaire: Université Claude Bernard Lyon I, 69622 Villeurbanne Cedex (FR)
(72) Inventeur: THIEULEUX, Chloé, F-69100 Villeurbanne (FR); BOUALLEG, Malika, F-69100 Villeurbanne (FR); CANDY, Jean-Pierre, F-69300 Caluire (FR); VEYRE, Laurent, F-38200 Jardin (FR); BASSET, Jean-Marie, F-69300 Caluire Et Cuire (FR)
(74) Mandataire: Sarlin, Laure V.
(86) Numéro de dépôt international: PCT/FR2009/051809
(87) Numéro de publication internationale: WO 2010/040926

(56) Documents cités:
- EP-A- 0 976 450
- WO-A-01/32558
- CA-A1- 2 499 782
- FR-A- 2 901 715
- BUDRONI ET AL: "Pd nanoparticles embedded in sponge-like porous silica as a Suzuki-Miyaura catalyst: Similarities and differences with homogeneous catalysts" JOURNAL OF CATALYSIS, ACADEMIC PRESS, DULUTH, MN, US, vol. 251, no. 2, 1 octobre 2007 (2007-10-01), pages 345-353, XP022282938 ISSN: 0021-9517
- APRILE C ET AL: "Synthesis and catalytic activity of periodic mesoporous materials incorporating gold nanoparticles" JOURNAL OF MATERIALS CHEMISTRY 20051107 ROYAL SOCIETY OF CHEMISTRY GB, [Online] vol. 15, no. 41, 7 novembre 2005 (2005-11-07), pages 4408-4413, XP002528288 Extrait de l'Internet: URL:doi:10.1039/b507418e> [extrait le 2009-05-14]
- LEE B ET AL: "Preparation of bicontinuous mesoporous silica and organosilica materials containing gold nanoparticles by co-synthesis method" MICROPOROUS AND MESOPOROUS MATERIALS, ELSEVIER SCIENCE PUBLISHING, NEW YORK, US, vol. 70, no. 1-3, 21 mai 2004 (2004-05-21), pages 71-80, XP004506225 ISSN: 1387-1811

## Description

La présente invention concerne le domaine technique des matériaux poreux structurés, utilisés notamment dans le domaine de la catalyse.

Les matériaux poreux structurés trouvent de nombreuses applications, notamment en tant qu'absorbants, catalyseurs, supports pour la catalyse, et plus récemment, dans les techniques de séparation, de diagnostic médical ou dans la micro-électronique.

De nombreux matériaux poreux structurés sont connus. A titre d'exemple, on peut notamment citer :
- les matériaux oxydes ou oxydes mixtes mésoporeux/microporeux, non cristallins et de porosité structurée, tels que ceux de la famille M41S (pour « Mesoporous Molecular Sieves », notamment décrits dans « A new family of mesoporous molecular sieves prepared with liquid crystal templates. », Beck, J. S.; Vartuli, J. C.; Roth, W. J.; Leonowicz, M. E.; Kresge, C. T.; Schmitt, K. D.; Chu, C. T. W.; Olson, D. H.; Sheppard, E. W. S. B. McCullen,t, J. B. Higgins, and J. L. Schlenkert., J. Am. Chem. Soc (1992), 114(27), 10834-43.) parmi laquelle les MCMs (pour « Mobil Corporation Materials »), la famille des HMS (Hexagonal Mesoporous Silica), des MSU (Michigan State University) ou des SBA (Santa Barbara) ;
- les matériaux macroporeux structurés ;
- les matériaux bimodaux structurés présentant deux types de porosité, notamment micro/mésoporeux ou méso/macroporeux.

Certains de ces matériaux sont utilisés en tant que catalyseurs hétérogènes qui sont le plus souvent à base ou constitués d'un oxyde métallique, oxyde de métalloïde, un oxyde mixte de métaux, métalloïdes ou métal/métalloïdes ou d'un mélange des dits oxydes, au sein duquel sont incorporées des particules métalliques, et en particulier des particules de métal 0, tel que le platine, le ruthénium, le nickel, l'or ou l'argent. A titre d'exemples de tels catalyseurs, on peut citer les catalyseurs contenant du Platine et supportés sur MCM-41, sur matrices mésoporeuses ou microporeuses ou sur zéolithes pour des réactions catalytiques diverses telles que la réduction des oxydes d'azotes NOx, la combustion des alcanes légers (C2 à C4), hydroisomérisation...(ref : « Platinum catalysts supported on macrostructured MCM - 41 for the selective catalytic reduction of lean NOx with hydrocarbons » Park, J.-L; Yun, J.-S.; Jeong, K.-E.; Ihm, S.-K.Studies in Surface Science and Catalysis, 170B, 1362-1367 (2007) ; «Surface properties of platinum catalysts based on various nanoporous matrices» Sobczak, Izabela; Grams, Jacek; Ziolek, Maria.Microporous and Mesoporous Materials, 99(3), 345-354 (2007); «The origin of the enhanced activity of Pt/ zeolites for combustion of C2-C4 alkanes» Garetto, T. F.; Rincon, E.; Apesteguia, C. R. Applied Catalysis, B: Environmental, 73(1-2), 65-72 (2007); « Hydroisomerization of a refinery naphtha stream over platinum zeolite -based catalysts » Ramos, Maria Jesus; Gomez, Juan Pedro; Dorado, Fernando; Sanchez, Paula; Valverde, Jose Luis, Chemical Engineering Journal, 126(1), 13-21 (2007) ; « Development of a simple method for the preparation of novel egg-shell type Pt catalysts using hollow silica nanostructures as supporting precursors »Wang, Jie-Xin; Chen, Jian-Feng, Materials Research Bulletin, 43(4), 889-896 (2008); « Toward a molecular understanding of shape selectivity » Smit, Berent and Maesen Theo L.M.Nature, 451, 671-678 (2008)).

Les matériaux structurés et mésoporeux tels que les M41S sont, notamment, obtenus par le procédé nommé LCT (de l'anglais "Liquid Crystal Templating" ou de "texturation par cristaux liquides") qui consiste à former une matrice minérale telle qu'un gel de silice ou d'aluminosilicate en présence de composés amphiphiles de type tensioactifs. Ce procédé met en oeuvre un procédé classiquement nommé procédé sol-gel. Schématiquement, la structure du gel initialement adoptée par les molécules de tensioactif imprime à la matrice minérale sa forme finale. Il semblerait, qu'au sein du gel, les précurseurs minéraux se localisent sur les parties hydrophiles des composés amphiphiles avant de se condenser entre eux, ce qui confère à la matrice minérale obtenue *in fine* un agencement spatial calqué sur celui du cristal liquide. Par élimination du tensioactif, notamment par traitement thermique, on obtient un matériau structuré mésoporeux, dont la structure est déterminée par l'empreinte de la structure cristal liquide initiale (C. T. Kresge, et al., Nature 1992, 359, 710-712 et D. Zhao, et al., Science 1998, 279,548).

De nombreux travaux portent sur l'incorporation de particules métalliques dans des structures poreuses à base d'oxyde. En particulier, des techniques mettant en oeuvre des réactions d'échange d'ions métalliques et de réduction in situ du métal (a- Goguet, A. et al., J. Catal. 2003, 220, 280-290. b- Chytil, S. et al, Topics in Catalysis 2007, 45, 93-99) ou encore une déposition de vapeur de composés métalliques suivie de leur décomposition (Benesis, A .H. et al. J. Catal. 1968, 10, 328) ou encore des techniques d'imprégnation (Konya, Z. et al. Catal. Lett. 2007, 113, 19-28).

Certes, ces techniques permettent d incorporer des particules de différents métaux au sein de supports poreux, mais ne se montrent pas satisfaisantes en termes de contrôle de la taille des particules, ou de contrôle de la distribution des particules métalliques au sein du matériau. Ce défaut de contrôle entraine notamment un frittage prématuré des particules lors du traitement thermique, ou encore dans le cadre des applications en catalyse, une réduction de l'activité du catalyseur.

D'autres travaux (Yang C.M., Lin H.A., Zibrowius B. et al. Chem. Mater. 19, 2007, 13, 3205) traitent de la décomposition de sels de palladium sélectivement dans les micropores de matériaux micro et mésoporeux, grâce au greffage préalable de ligands organiques dans la microporosité. Ceci est rendu possible en réalisant des traitements de surface des mésopores pour éviter le plus possible le greffage dans les mésopores. Cette méthode permet de générer des nanoparticules dans un support préexistant. Aussi, les particules sont environ de la taille des micropores du matériau, c'est à dire de taille inférieure à 2 nm.

D'autres travaux antérieurs ont décrit des procédés permettant d'intégrer des agrégats ou particules métalliques, dans un matériau, mais dans ces cas, les particules sont soit intégrées dans les pores du matériau inorganique obtenu, soit le matériau inorganique obtenu n'est pas organisé.

Le document CA 2 499 782, par exemple, décrit un procédé de préparation d'un matériau silicate poreux par condensation d'un silicate qui fonctionne comme précurseur, en présence d'un ligand pour un métal. Dans ce document, le matériau silicate fonctionnalisé est obtenu en amont de l'ajout de particules métalliques. L'exemple 6 du document CA 2 499 782, néanmoins, prévoit de faire croitre un matériau autour de nanoparticules stabilisées avec du MPTMS qui est un ligand qui comporte une fonction thiol. Mais, les particules de palladium utilisées dans ce procédé sont des agrégats de grande taille et de taille non homogène. Compte tenu de la taille des particules mises en oeuvre, ces dernières ne peuvent pas être positionnées dans les murs du matériau inorganique obtenu.

Le document FR 2 901 715, quant à lui, décrit un procédé de préparation d'un nano-matériaux hybrides contenant des nanoparticules métalliques, éventuellement oxydées, comprenant :
1) la formation de nanoparticules dudit métal, à partir d'un précurseur dudit métal, en présence d'un ligand de formule (L) :

   Y-X-G (L)

   sous pression d'hydrogène ;
2) la polymérisation du ligand (L) au sein d'un matériau solide, et
3) la calcination du matériau obtenu ;

Il est précisé que la partie G représente un groupe hydrolysable. Les ligands utilisés dans les exemples sont des ligands échangeables. De plus, dans ce document, le matériau obtenu est non structuré, étant donné qu'il n'est pas prévu d'utiliser un agent porogène. D'ailleurs, la quantité de matériau inorganique, par rapport aux particules métalliques est très faible, à l'exemple 1 du document FR 2 901 715 notamment.

Aprile Carmela et al. dans J. Mater. Chem., 2005, 15, 4408-4413, quant à eux, décrivent un procédé qui met en oeuvre des nanoparticules d'or stabilisées, en présence d'un ligand hydrophile de type cetyltriméthylammoniumtrialkoxysilane (CTA⁺), et la synthèse du matériau est réalisée, en mettant en présence du TEOS avec des précurseurs organiques silylés, et du bromure d'hexadécyltriméthyammonium en tant qu'agent structurant. Des ligands de type silane sont donc utilisés, mais, la partie (alcoxy)silane n'est pas située côté nanoparticules et ne permet donc pas d'avoir un ligand non échangeable. D'ailleurs, page 4411, il est indiqué que les nanoparticules métalliques sont situées dans les pores du matériau obtenu. On peut encore citer la publication de Gerolamo Budroni et al. dans Journal of Catalysis 2007, 251, 345-353 qui décrit un procédé proche dans lequel aucun agent porogène n'est utilisé, de sorte que le matériau obtenu n'est pas structuré.

La demande WO 01/32558 propose des matériaux intégrant des particules au sein des murs de structures mésoporeuses. Néanmoins, seules des particules d'oxyde, d'hydroxyde ou des oxyhydroxydes de métaux très difficilement voire non réductibles en métal 0, sont incorporées au sein de matériaux. Ces particules incorporées dans des matrices inorganiques ont pour but d'en améliorer la cristallinité et de conférer des propriétés physico/chimiques différentes (adsorption, résistance mécanique, oxydo/réduction notamment pour la photo-catalyse et la catalyse..), mais elles ne peuvent en aucun cas permettre de réaliser des réactions catalytiques comme le font les particules de métal 0, telles que les réactions d'hydrogénation sélective (des alcynes en alcènes et des alcènes en alcanes...) les réactions de deshydrogénation des hydrocarbures, l'hydrogénolyse d'hydrocarbures, la réaction de Fischer-Tropsch ou la réaction d'hydrodéchloration (du chlorobenzène en benzène...)...

De plus, le problème majeur en catalyse hétérogène est la désactivation des catalyseurs. Cette perte d'activité, due à un empoisonnement du catalyseur, par exemple par des résidus carbonés, au cours du temps nécessite un traitement à haute température, par exemple sous courant de gaz oxydant, pour régénérer le catalyseur. Or, ce traitement conduit très souvent au frittage des particules contenues dans le solide. Ce problème de frittage n'est, à ce jour, que partiellement résolu. Pour l'éviter au maximum, des dopants sont ajoutés pour stabiliser les dites particules, mais celles-ci sont, de ce fait, beaucoup moins actives, lors de la réaction catalytique recherchée.

Dans ce contexte, un des objectifs de l'invention est de proposer un nouveau procédé de préparation de matériaux poreux structurés permettant de localiser à façon, un ou plusieurs types de particules métalliques, dans une charpente inorganique poreuse structurée, ce procédé se devant d'être facile à mettre en oeuvre.

Un autre objectif de l'invention est de fournir un procédé conduisant à des matériaux poreux structurés incorporant des particules métalliques qui soient particulièrement stables et dans lequel les particules métalliques soient réactives et accessibles, du fait de leur répartition régulière et de leur localisation.

Aussi, la présente invention a pour objet un procédé de préparation d'un matériau poreux structuré comportant une charpente inorganique structurée composée de murs à base d'oxyde métallique dans lesquels des particules de métal 0 sont incorporées, qui comporte les étapes suivantes :
- disposer d'une suspension de particules hydrophiles de métal 0 stabilisées par des ligands non échangeables qui confèrent leur caractère hydrophile aux particules,
- faire croitre la charpente inorganique à partir d'un précurseur inorganique, autour des particules de métal 0 stabilisées par les ligands non échangeables qui confèrent leur caractère hydrophile aux particules, en présence d'un agent porogène,
- éliminer l'agent porogène et au moins partiellement les ligands non échangeables qui confèrent leur caractère hydrophile aux particules.

Les matériaux susceptibles d'être obtenus par un tel procédé font également partie intégrante de l'invention.

La description ci-après, en référence aux Figures annexées permet de mieux comprendre l'invention.
La **Figure 1** présente schématiquement les étapes successives du procédé selon l'invention.
Les **Figure 2, 3** et **4** représentent les histogrammes de la taille de particules obtenues.
La **Figure 5** présente le diffractogramme sur poudre des Rayons X aux petits angles d'un matériau obtenu selon le procédé de l'invention.
Les **Figures 6A** et **6B** sont des images par microscopie électronique en transmission d'un matériau obtenu selon le procédé de l'invention,
La **Figure 7** présente le spectre WAXS d'un matériau obtenu selon le procédé de l'invention.
La **Figure 8** présente le diffractogramme sur poudre des Rayons X aux petits angles d'un matériau obtenu selon le procédé de l'invention.
La **Figure 9** présente en trait plein, les transformées de fourrier expérimentales avant calcination et après calcination d'un matériau obtenu selon le procédé de l'invention et les modèles théoriques d'une cristallite de Pt de 2 nm en réseau cristallographique de type cubique face centrée (fcc).
La **Figure 10** présente différentes images par microscopie électronique en transmission d'un matériau obtenu selon le procédé de l'invention.
La **Figure 11** montre le taux de conversion du propène en propane en fonction du temps obtenu avec un matériau obtenu selon le procédé de l'invention.
La **Figure 12** présente l'isotherme d'adsorption/désorption d'azote à -196°C (77K) d'un matériau obtenu selon le procédé de l'invention.
La **Figure 13** présente le spectre XPS d'un matériau obtenu selon le procédé de l'invention.
La **Figure 14** présente une image par microscopie électronique en transmission d'un matériau obtenu selon le procédé de l'invention.
La **Figure 15** présente une image par microscopie électronique en transmission d'un matériau obtenu selon le procédé de l'invention.
Les **Figures 16a****)** et **b)** présentent respectivement l'isotherme d'adsorption/désorption d'azote à -196°C (77K) et la distribution poreuse d'un matériau obtenu selon le procédé de l'invention.
La **Figure 17** compare les taux de conversion obtenus par hydrogénation du propène en réacteur dynamique, avec un matériau selon l'invention, et un matériau avec des particules de Pt dans les pores.
La **Figure 18** présente l'isotherme d'adsorption/désorption d'azote à -196°(77K) d'un matériau obtenu selon le procédé de l'invention.
La **Figure 19** présente une image par microscopie électronique en transmission d'un matériau obtenu selon le procédé de l'invention.
La **Figure 20** compare les taux de conversion obtenus pour l'hydrogénation du propène avec un catalyseur de référence et un matériau selon l'invention.
La **Figure 21** présente les quantités de styrène et d'éthylbenzène en fonction du temps, obtenues lors de l'hydrogénation du styrène avec un matériau selon l'invention.
La **Figure 22** présente les quantités de styrène et d'éthylbenzène en fonction du temps, lors de l'hydrogénation du styrène avec un catalyseur de référence Pt sur alumine.
La **Figure 23** présente, à titre comparatif, une image par microscopie électronique en transmission d'un matériau contenant des particules de Pt hydrophiles stabilisées initialement par un ligand hydrophile échangeable de type diol.

Dans le cadre du procédé selon l'invention, la croissance de la charpente inorganique du matériau poreux structuré est directement réalisée autour de particules métalliques existantes. On obtient ainsi une répartition régulière des particules métalliques qui sont bien espacées et réparties au sein du matériau obtenu. Le procédé selon l'invention permet d'éviter l'agglomération des particules métalliques et conduit ainsi à une bonne structuration du matériau, par rapport aux techniques antérieures. Aussi, au sein du matériau obtenu, les particules sont de petite taille et sont bien réparties. Les particules présentes au sein du matériau ont une taille nanométrique, c'est-à-dire, notamment, que le coeur métallique (hors ligands) est sphérique et qu'au moins, pour 50% de la population des nanoparticules, le coeur métallique présente un diamètre moyen de 1 à 10 nm, le diamètre moyen étant déterminé, par exemple, par microscopie électronique à transmission sous forme d'un histogramme de tailles ou, de préférence, par la technique de diffraction des Rayons X aux grands angles (WAXS de l'anglais « Wide Angle X ray Scattering »). Le matériau obtenu est particulièrement stable, même lorsqu'il subit un traitement thermique, la taille des pores et des particules métalliques restant inchangée après traitement.

Grâce au procédé selon l'invention, les particules sont régulièrement réparties, ce qui limite leur frittage, et sont stabilisées par la charpente poreuse inerte, ce qui limite également leur frittage, tout en les laissant parfaitement accessibles et réactives.

Le procédé selon l'invention conduit à un matériau structuré poreux. Par « matériau structuré », on entend un matériau qui présente une structure organisée, notamment caractérisée par la présence d'au moins un pic de diffraction dans un diffractogramme sur poudre des Rayons X aux petits angles (Small Angle X-Rays Scattering (Glatter et Kratky, Academic Press London-1982)). Le pic de diffraction observé dans le diffractogramme sur poudre des Rayons X aux petits angles obtenu pour un matériau structuré est associé à une distance de répétition caractéristique du matériau considéré. Cette distance de répétition est également appelée « période spatiale de répétition du système structuré » et correspond à la périodicité des pores au sein du matériau, dans le cas d'un matériau poreux. Dans le matériau selon l'invention, la charpente est donc structurée, c'est pourquoi, on parle de murs et de pores.

Le matériau obtenu grâce au procédé selon l'invention est poreux, la taille des pores étant fonction de l'agent porogène utilisé. En particulier, le matériau obtenu est microporeux, mésoporeux ou présente une double porosité micro/méso. On entend par microporeux, un matériau présentant des pores d'une taille inférieure à 2 nm et par mésoporeux, un matériau présentant des pores d'une taille comprise entre 2 et 50 nm. La texture d'un matériau (à savoir la surface spécifique, le type de pores et leur taille ainsi que le volume poreux) est obtenue par adsorption/désorption d'azote à - 196°C (77K).

La charpente inorganique du matériau obtenu dans le cadre de l'invention est constituée d'un oxyde métallique. Le terme « oxyde métallique » est utilisé au sens large, dans le cadre de l'invention et inclut notamment les oxydes de métaux, les oxydes de métalloïdes, les oxydes mixtes de métaux et/ou métalloïdes.

A titre d'exemples de matériaux poreux structurés selon l'invention, on peut citer les structures poreuses composées d'au moins un oxyde métallique des groupes 3 à 11, ou d'au moins un oxyde de métalloïde d'un élément des groupes 2 et 12 à 14, ou d'un oxyde mixte de différents métaux ou métalloïdes ou d'un mélange de ces oxydes. Notamment, on peut citer les oxydes de silicium, aluminium, titane, étain, tantale ou zirconium. Les charpentes de silice ou d'oxydes mixtes, silice/oxyde de titane ou silice/alumine (également nommé aluminosilicates), sont particulièrement préférées.

La structuration du matériau final pourra être de type vermiculaire, lamellaire, hexagonal (1D ou 2D) ou cubique avec une préférence pour la structuration hexagonale.

Le matériau obtenu présente, de préférence, une surface spécifique de 20 à 1200 m²/g et préférentiellement de 300 à 1100 m²/g dans le cas de charpente composée majoritairement de silice. La surface spécifique est notamment déterminée par mesure d'adsorption désorption d'azote selon la méthode décrite ci-après dans les méthodes de caractérisation.

La charpente est réalisée, en présence d'au moins un agent porogène, du type tensio-actif notamment, *in situ,* autour de particules métalliques hydrophiles du fait de la présence de ligands non-échangeables choisis à la fois pour leur conférer leur caractère hydrophile et assurer leur stabilisation. La nature non échangeable des ligands, ajouté au fait qu'ils rendent les particules hydrophiles permet aux particules d'être localisées, dans le matériau final dans les murs et non dans les pores de la structure poreuse. Il est important que les ligands soient non-échangeables, afin que les particules métalliques conservent leur caractère hydrophile. Par « non-échangeables », on entend notamment que les ligands conférant le caractère hydrophiles aux particules ne doivent pas s'échanger avec les agents porogènes. En effet, un tel échange avec des tensio-actifs jouant le rôle d'agent porogène, aurait pour effet de rendre les particules métalliques hydrophobes et ces dernières viendraient alors se placer dans les pores du matériau et non dans les murs de la charpente.

Les ligands utilisés confèrent un caractère hydrophile aux particules, du fait de la présence de groupements polaires ou polarisables. Ces ligands présentent donc un caractère hydrophile, lorsqu'ils sont sur la particule et sont nommés ligands hydrophiles dans la suite de la description. Par « groupements polaires », on entend un groupement qui présente un moment dipolaire. Par « groupements polarisables », on entend un groupement qui se polarise (i.e. qui présentera un moment dipolaire) dans des conditions spécifiques (comme par exemple dans un solvant avec une constante diélectrique élevée). A titre d'exemple de groupements polaires ou polarisables, on peut notamment citer les atomes d'halogène, tel que le chlore, les groupements amine, ammonium, phosphonate, phosphonium, hydroxyde, thiol, sulfonate, nitrate, carbonate, alcool ... Comme groupes amine particulièrement adaptés, on peut citer les imidazoles, les sels d'imidazolium et les sels d'alkyltriméthylammonium.

Le caractère non échangeable du ligand peut notamment être assuré par la présence d'un atome de silicium, d'étain ou de germanium, jouant le rôle de point d'ancrage du ligand sur la particule métallique. Aussi, les ligands de type silane ou dérivés stanneux (ou stanniques) sont préférés, de par leur synthèse plus aisée. Les ligands utilisés dans le cadre de l'invention présentent différents avantages par rapport aux ligands utilisés dans l'art antérieur qui comprennent une fonction thiol qui peut conduire à une liaison stable avec certaines particules. En effet, les ligands de type thiol ne sont pas compatibles avec de nombreuses réactions catalytiques, pour lesquelles ils se comportent comme des poisons. Par contre, de façon inattendue, les ligands utilisés dans le cadre de l'invention sont tout à fait compatibles avec l'utilisation des matériaux obtenus en catalyse. Notamment, il a été constaté qu'un matériau obtenu grâce au procédé selon l'invention qui contient des nanoparticules de Pt (comme démontré dans les exemples ci-après) est actif en hydrogénation du propène : les nanoparticules métalliques du matériau sont donc accessibles et réactives. De plus, les TOF calculés pour un catalyseur de référence contenant des nanoparticules de platine « nues » (i.e. sans ligand de surface) et pour le matériau selon l'invention sont très similaires : les nanoparticules de Pt contenues dans les murs de notre matériau sont donc aussi accessibles et réactives que des particules de Pt « nues ». Les nanoparticules de Pt ne sont donc pas empoisonnées par la présence d'atomes de Si à leur surface.

A titre d'exemple de ligands hydrophiles non-échangeables conférant leur caractère hydrophile aux particules qui peuvent être mis en oeuvre dans le cadre de l'invention, on peut citer, le 3-chloropropylsilane, le N-(3-trihydrogenosilylpropyl)-imidazole, le chlorobenzylsilane, le chlorodiméthylsilane, les sels de N-(3-trihydrogenosilylpropyl)alkylimidazolium ou les sels de N-(3-trihydrogenosilylpropyl)arylimidazolium, N-(benzyltrihydrogenosilyl)-imidazole, les sels de N-(benzyltrihydrogenosilyl)-alkylimidazolium ou les sels de N-(benzyltrihydrogenosilyl)-arylimidazolium, et aussi les sels de N-(benzyltrihydrogenosilyl)trialkylammonium ou le dibutyl-4,7,10-trioxaundécylstannane.... De tels ligands sont commerciaux, ou pourront être préparés selon des techniques bien connues de l'homme de l'art. Dans le cas de ligands comprenant un atome d'étain ou de germanium, on pourra se référer **à** F. Ferkous, Journal of Organometallic Chemistry, 1991,Volume 420, Issue 3, Pages 315-320 et à P.Riviere, Journal of Organometallic Chemistry, 49 (1973) 173-189.

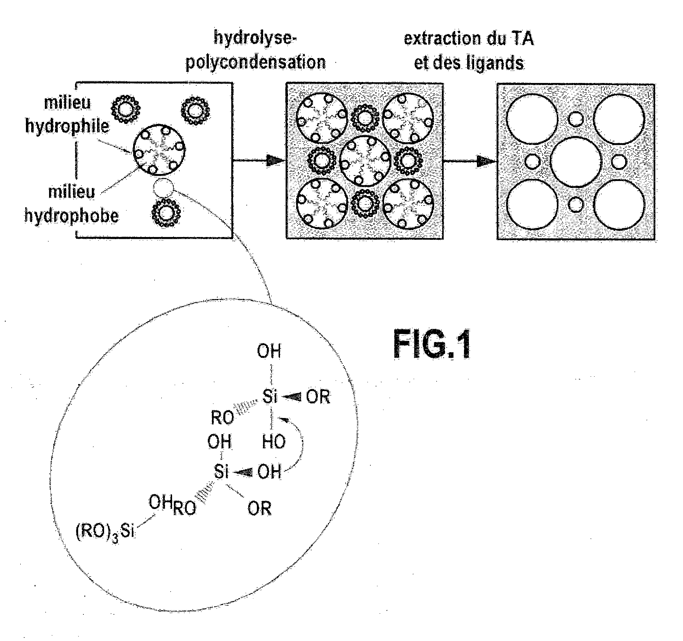

Les étapes successives du procédé selon l'invention sont présentées **Figure 1****.** La première étape du procédé selon l'invention consiste à disposer d'une suspension colloïdale de particules métalliques hydrophiles et stabilisées par des ligands non-échangeables. De telles suspensions sont préparées selon des techniques bien connues de l'homme de l'art.

Notamment, un précurseur métallique, classiquement utilisé dans la synthèse de particules du métal souhaité est mis en présence des ligands hydrophiles non-échangeables comportant un groupe polaire ou polarisable dans un solvant organique classique polaire (eau, alcool, THF, Ether..) ou apolaire (hydrocarbures saturés ou insaturés), le THF étant particulièrement préféré. De préférence, la synthèse des particules métalliques est réalisée sous pression d'hydrogène ou en présence d'un réducteur (comme NaBH₄) avantageusement avec 0,2 à 5 équivalents de ligands stabilisateurs par atome de métal engagé. A titre d'exemple de précurseurs métalliques, on peut citer le Ru(COD)(COT), Pt(dba)₂, Ni(COD)₂, HAuCl₄... avec dba = dibenzylidène acetone, COD = cyclooctadiène et COT = cyclooctatriène.

Les particules métalliques peuvent notamment être des particules de platine, ruthénium, or, nickel, cobalt, fer, argent, palladium ou rhodium.

Les particules obtenues et utilisées dans le cadre de l'invention sont de taille nanométrique, c'est-à-dire que le coeur métallique (hors ligands) est, de préférence, sphérique et qu'au moins, pour 50% de la population des nanoparticules, le coeur métallique présente un diamètre moyen de 1 à 10 nm, le diamètre moyen étant déterminé, par exemple, par microscopie électronique à transmission sous forme d'un histogramme de tailles ou, de préférence, par la technique de diffraction des Rayons X aux grands angles (WAXS de l'anglais « Wide Angle X ray Scattering »). Les particules métalliques sont avantageusement monodisperses, c'est-à-dire qu'elles présentent une distribution de taille très étroite autour d'une valeur moyenne et notamment 50% des particules ont pour taille qui correspond à la taille moyenne ± 0,5 nm, déterminée par microscopie électronique à transmission sous forme d'un histogramme de tailles. La taille des particules en suspension correspond à la taille des particules présentes dans le matériau obtenu, le procédé selon l'invention n'entrainant aucune variation de taille.

La seconde étape consiste à faire croitre la structure poreuse du matériau, autour des particules métalliques en suspension dans un solvant approprié, en présence d'un agent porogène, pour conférer la porosité souhaitée. Les particules métalliques viennent se localiser au sein même de la structure constituant la charpente du matériau et non pas à l'intérieur des pores. Les particules métalliques sont donc totalement emprisonnées physiquement dans les murs de la charpente du matériau.

Le précurseur minéral utilisé est, par exemple, un alcoxyde ou hydroxyde de métal ou métalloïde parmi lesquels seront préférés les silicates ou les tétraalcoxysilane, tétraalcoxyde de titane ou d'aluminium. De manière classique, la croissance de la charpente métallique est réalisée par procédé sol-gel (L.L. Hench et al. Chem. Rev. 1990, 33-72 et S. Biz et al. Catal. Rev. - Sci. Eng 1998, 0(3). 329-407).

Notamment la croissance de la charpente métallique est réalisée dans un milieu aqueux ou aqueux en mélange avec au moins un co-solvant de type alcool (de préférence des alcools linéaires : butanol...), de type éther (de préférence THF) ou le diméthylformamide (DMF)

La croissance de la charpente sera, de préférence, réalisée avec au moins une des conditions suivantes, seules ou de préférence en combinaison :
- une température de 0°C à 100°C préférentiellement de 20°C à 65°C,
- un ratio métal du précurseur inorganique/ agent porogène en moles = 30-300,
- un ratio métal des particules/métal du précurseur inorganique en poids de 0,001 à 50% ou encore inférieur à 10%, et de préférence de 0,001 à 5% et préférentiellement de 0,005 à 5%, ou encore de 0,05 à 5%,
- un pH de 0 à 10 et préférentiellement de 0 à 4,
- en présence d'un catalyseur d'hydrolyse-polycondensation du type acide, basique ou nucléophile et préférentiellement tel que l'HCl (acide), NH₃ ou KOH, NaOH (basiques) ou NaF ou TBAF (nucléophiles).

Il est également possible de réaliser, avant l'ajout du précurseur inorganique nécessaire à la croissance de la charpente, une suspension colloïdale de particules de métal 0 stabilisées et rendues hydrophiles par des ligands non échangeables, additionnée de l'agent porogène, de préférence dans un mélange eau/THF. Pour cela, l'agent porogène sera ajouté à la suspension colloïdale de particules métalliques précédemment formée ou inversement.

La croissance du matériau poreux autour des particules d'oxydes métalliques est réalisée, en présence d'un agent porogène également nommé templates ou tensio-actifs. Le plus souvent, les interactions entre le tensioactif et les précurseurs minéraux seront électrostatiques ou de Van Der Waals. L'agent porogène présent dans le mélange réactionnel est un composé amphiphile de type tensioactif, notamment un copolymère. La caractéristique essentielle de ce composé est qu'il est susceptible de former des micelles dans le mélange réactionnel, de façon à mener, par mise en oeuvre du mécanisme coopératif de texturation défini précédemment, à la formation ultérieure, d'une matrice minérale possédant une structure organisée. On peut notamment citer, à titre d'exemples de telles molécules organiques, pouvant être utilisées comme agent porogène :
- les templates anioniques, tel que le dodécyl sulfate de sodium;
- les templates cationiques tels que les sels d'ammonium et notamment les sels de tétraalkylammonium comme ceux de cétyltrialkylammonium ou dodecyltrialkylammonium, les sels d'imidazolium tels que le bromure de 1-hexadécane-3-méthylimidazolium, les sels de pyridinium tel que le chlorure de n-hexadécylpyridinium;
- les templates non-ioniques, et notamment les amines, telles que l'hexadécylamine ou la dodecylamine;
- les oxydes d'alkylpolyéthylène ou d'alkylarylpolyéthylène, tels que le Brij® 52 (C₁₆H₃₃O(CH₂CH₂O)₂H), le Tergitol® 15-S-12 (C11-15H23-31O(CH2CH2O)12H), le Triton X® 25-100 (C₁₄H₂₂O(C₂H₄O)ₙ₁ avec n1=9-10), le Montanox® 20 (Sorbitan.20EO.monooléyle ester), l'octylphénol-10 EO (p-C₈H₁₇C₆H₄O(CH₂CH₂O)₁₀H), le lauryl éther-n EO (C₁₂H₂₅O(CH₂CH₂O)ₙ₂H, avec n₂∼2,4,8) ;
- les templates de type polysorbate, tels que le Tween® 20 (nom IUPAC : polyoxyéthylène (20) sorbitan monolaurate) et 30 ;
- les copolymères à blocs amphiphiles, tels que les copolymères triblocs Pluronic® P123 (EO₂₀-PO₇₀-EO₂₀), le Pluronic® F127 (EO₇₇-PO₂₉-EO₇₇) ou le Pluronic® F108 (EO₁₃₂-PO₅₀-EO₁₃₂) ;
- les polymères (fonctionnels ou non) et notamment les polymères à blocs tels que le Pluronic® P123, le Pluronic® F127 ou F108 cités précédemment ou encore les polymères classiques du type PE, PP, PMMA, polystyrène....

De tels agents porogènes ont déjà été largement utilisés dans l'art antérieur. Dans le cadre de l'invention, on choisira de préférence des conditions expérimentales (nature et taille de l'agent porogène, pH de la synthèse, le rapport agent porogène/précurseur minéral, température, type de catalyseur d'hydrolyse-polycondensation) pour permettre l'obtention de murs de taille suffisante, c'est-à-dire d'épaisseur suffisante, pour pouvoir y insérer les particules métalliques souhaitées. En effet, les différents essais réalisés par les inventeurs ont montré que, pour que les particules métalliques viennent se loger de façon appropriée au sein des murs du matériau, il était essentiel que la taille des particules rendues hydrophiles et stabilisées par les ligands non échangeables soit inférieure ou égale à la taille des murs. Ainsi, les particules peuvent être totalement intégrées à l'intérieur des murs de la charpente. La taille des particules stabilisées par les ligands non échangeables est déterminée à partir de la taille moyenne des particules donnée par microscopie électronique à transmission sous forme d'un histogramme de tailles ou, de préférence, par la technique de diffraction des Rayons X aux grands angles (WAXS) et modélisation de l'encombrement des ligands en utilisant les longueurs des liaisons et les angles entre les atomes. Aussi, de façon avantageuse, les conditions du procédé seront choisies dans ce but, et correspondront aux conditions précédemment citées qui permettent d'atteindre un tel résultat.

La taille des murs est, avantageusement, supérieure à 3 nm et préférentiellement comprise dans la gamme allant de 5 à 15 nm. La taille des murs est notamment déterminée en utilisant la diffraction des rayons X aux petits angles et les mesures d'adsorption désorption d'azote selon les méthodes décrite ci-après dans les méthodes de caractérisation.

Dans certains cas, il pourra être souhaitable de faire croitre la charpente inorganique, autour d'une solution colloïdale d'un mélange de particules métalliques de nature différente :
- par exemple un mélange de particules métalliques d'un métal donné rendues hydrophiles et stabilisées par la présence de ligands non-échangeables assurant leur hydrophilie et de particules métalliques d'un autre métal, rendues hydrophiles et stabilisées également par des ligands non-échangeables assurant leur hydrophilie. Dans ce cas, le matériau comportera différents types de particules métalliques dans les murs.
- par exemple un mélange de particules métalliques d'un métal donné rendues hydrophiles et stabilisées par la présence de ligands non-échangeables assurant leur hydrophilie et de particules métalliques hydrophobes d'un autre métal, stabilisées par des ligands hydrophobes. Dans ce cas, les particules hydrophiles se positionneront dans les murs et les particules hydrophobes dans les pores.

S'il est souhaité d'avoir également des particules hydrophobes dans les pores, l'agent porogène sera avantageusement choisi pour conduire à des tailles de pores suffisamment grandes pour accueillir au moins un type de particule métallique stabilisée par des ligands hydrophobes. Dans ce cas, le ou les agents porogènes seront choisis parmi la famille des copolymères blocs et préférentiellement les polymères triblocs de type pluronic® P123, F127, F108 et P104.

La mise en oeuvre de différents types de particules dans les murs, ou dans les pores et dans les murs, est particulièrement avantageuse pour des applications en catalyse en cascade, ou bifonctionnelle. Il est également possible que le métal de l'un des types de particules, notamment localisées dans les murs, soit de nature magnétique, tel que le nickel ou le fer, pour faciliter la séparation notamment.

Dans le cas où des particules métalliques stabilisées par des ligands hydrophobes sont également utilisées, on pourra utiliser des ligands comportant des groupes de type silane, stanneux ou thiol (SiHₓ ,SnHy ou SH pour stabiliser les particules).

A titre d'exemple de tels ligands hydrophobes, on peut citer les alkylsilanes, arylsilanes ou alkylétain tels que le n-butyltrihydrogénosilane, le n-octyltrihydrogénosilane, le phényltrihydrigénosilane, le benzyltrihydrogénosilane ou le tributylhydrogénoétain et le triméthylhydrogénoétain, alkylthiol tel que le butylthiol.

L'étape de croissance du matériau est suivie d'un traitement destiné à éliminer l'agent porogène, et ainsi libérer la porosité du matériau. La partie organique des ligands utilisés est également éliminée. Dans le cas de ligands silane ou stanneux, les atomes de Si ou Sn sont, par contre, conservés, c'est pourquoi on parle d'élimination partielle des ligands non échangeables conférant leur caractère hydrophile aux particules. Il en va de même avec les ligands contenant un atome de germanium qui lui aussi conservé lors du traitement. Seule la partie organique des ligands est éliminée. Un tel traitement peut être réalisé par traitement thermique de type calcination. La température finale de calcination peut aller jusqu'à 500° C et sera préférentiellement de l'ordre de 350° C. On pourra utiliser un profil de montée en température compris entre 0,2°C par minute et 3°C par minute, et de préférence suivant un profil de montée en température compris entre 0,5°C par minute et 2°C par minute, pourra être mise en oeuvre.

Il est également possible d'éliminer les agents porogènes et les ligands utilisés, par dégradation en milieu aqueux sous irradiation UV, en présence d'un sel métallique. Les conditions d'un tel traitement sont très douces :
- Le sel métallique qui peut être utilisé pourra être un sel organique ou inorganique. A titre d'exemple de sels organiques, on peut citer la famille des carboxylates, au sein de laquelle préférentiellement les citrates, les oxalates et les pyruvates. A titre d'exemple de sels inorganiques, on peut citer les sulfates, hypochlorates, chlorates, nitrates et carbonates. Différents sels métalliques peuvent convenir, en particulier, on utilisera les sels de titane, vanadium, chrome, manganèse, fer, cobalt ou nickel et, en particulier, les sels ci-dessus mentionnés. Les sels de fer sont particulièrement préférés. Le métal utilisé se trouve sous une forme qui va pouvoir être oxydée ou réduite, dans les conditions de traitement sous UV, puis qui va pouvoir respectivement être réduite ou oxydée.
- Le traitement est réalisé en milieu aqueux. Le milieu aqueux est, par exemple constitué d'eau, dans lequel le sel métallique utilisé est mis en solution ou suspension. Le pH de la solution est adapté au sel métallique utilisé et varie le plus souvent entre 2 et 8, de préférence entre 3 et 6. Le traitement sous UV peut être réalisé sous n'importe quel type d'atmosphère, contrôlée ou non. De préférence, le traitement est réalisé sous une atmosphère contenant de l'oxygène O₂ gazeux, par exemple sous air ou sous courant d'oxygène.

- L'élimination, par dégradation, de la partie organique, ne nécessite pas de traitement thermique à température élevée. Par exemple, le traitement sous UV est réalisé à une température comprise entre 0 et 100°C, de préférence entre 10 et 50 °C, préférentiellement entre 20 et 30°C, notamment à température ambiante (environ 25°C). Les temps de traitement sont relativement courts, et varient par exemple de 1 à 12 heures, de préférence de 3 à 8 heures.
- L'irradiation sous UV peut couvrir une plage de longueurs d'onde correspondant aux UV-A (400-315 nm), UV-B (315-280 nm) et UV-C (280-10 nm) ou aux UV-A seuls. Pour cela, une lampe UV traditionnelle, avec ou sans filtre, pourra être utilisée (400 nm-10 nm).
- La quantité de sel métallique correspond à une quantité catalytique par rapport à la réaction de dégradation de la molécule organique envisagée. Afin que le temps de traitement soit court, le rapport molaire (sel métallique/ molécule à dégrader) envisagé peut varier par exemple de 0,01 à 3, de préférence de 0,1 à 1,5.

Un tel traitement par dégradation sous UV, tout comme le traitement thermique, est non destructeur de la partie inorganique restante et n'endommage pas la structure du matériau traité, ni même les particules métalliques.

Il apparaît donc clairement que le procédé selon l'invention permet de préparer des matériaux poreux structurés, et notamment des matériaux mésostructurés, contenant des particules, et en particulier des nanoparticules métalliques localisées sélectivement au sein des murs de la charpente poreuse. Le procédé selon l'invention conduit à l'obtention de particules régulièrement distribuées dans le solide. Ces particules sont complètement accessibles et réactives. En outre, elles sont, de par leur localisation à l'intérieur des murs et leur distribution régulière, stabilisées vis-à-vis de traitements thermiques, c'est-à-dire qu'il y a peu ou pas de frittage et pas de lixiviation. Le procédé selon l'invention est notamment très avantageux pour la préparation de catalyseurs hétérogènes robustes et stables, et bien plus stables, que ceux obtenus par les méthodologies classiques comme la décomposition de sels métalliques ou l'imprégnation de solutions colloïdales sur des supports poreux ou non poreux. Le procédé selon l'invention est, entre autre, parfaitement adapté pour i) la synthèse de matériaux mono- ou multi-métalliques, pouvant contenir plusieurs types de particules différentes, ii) le remplacement des catalyseurs hétérogènes existants, iii) la synthèse de nouveaux catalyseurs mono- ou multi-métalliques.

Les matériaux obtenus, dans le cadre de l'invention, contiennent de petites particules monodisperses en taille. Or, les techniques antérieures ont montré que, jusqu'à maintenant, il était très difficile de générer sur des supports de très petites particules monodisperses. De plus, dans le cadre de l'invention, il est possible de générer, à façon, des matériaux poreux structurés contenant plusieurs types de particules sans interaction des particules entre elles, ce qui était très difficile avec les techniques antérieures. En effet, le procédé selon l'invention permet de préparer des matériaux du type catalyseurs hétérogènes stables contenant un ou plusieurs métaux différents, sous la forme de particules de métal 0. La présence de particules de métaux différents permet notamment d'utiliser les matériaux préparés, en catalyse bifonctionnelle ou pour des réactions en cascade.

Dès lors, le procédé selon l'invention ouvre de nouvelles perspectives à la catalyse hétérogène, en permettant la préparation de différents matériaux multi-métalliques. Le procédé et les matériaux selon l'invention sont donc plus particulièrement intéressants pour le domaine de la catalyse hétérogène. Cependant, dès lors que l'invention permet de localiser à façon des particules métalliques de natures diverses dans des charpentes poreuses, elle peut trouver une application dans les domaines de la purification des gaz ou de la microélectronique (pour l'obtention de mémoires magnétiques).

Les exemples ci-après permettent d'illustrer l'invention, mais n'ont aucun caractère limitatif. Les caractérisations sont réalisées dans les conditions suivantes :

### Analyse élémentaire.

Les analyses élémentaires ont été réalisées au "Laboratoire de Synthèse et Electrosynthèse Organométalliques", UMR 5188 CNRS, Dijon, France et au "Service Central d'Analyses" du CNRS à Vemaison, France.

### Microscopie électronique en transmission (TEM).

### Images de microscopie des solutions colloïdale:

Les images de microscopie ont été effectuées au "Centre Technologique des Microstructures", UCBL, Villeurbanne, France, en utilisant un microscope électronique en transmission, Philips 120 CX. La tension d'accélération était de 120 kV. Une goutte de la suspension colloïdale de Pt, préalablement diluée dans l'éthanol, a été déposée sur une grille en cuivre recouverte par un film de carbone.

### Images de microscopie des matériaux poreux contenant des nanoparticules de métal (0):

1) Les images de microscopie ont été effectuées au "Centre Technologique des Microstructures", UCBL, Villeurbanne, France, en utilisant un microscope électronique en transmission, Philips 120 CX. La tension d'accélération était de 120 kV. Les grilles ont été préparés soit i) en déposant une goutte d'une suspension du solide contenant des nanoparticules de Pt dilué dans l'éthanol, sur une grille en cuivre recouverte par un film de carbone soit ii) en déposant une fine coupe (50-70 nm) préparé par ultramicrotomie du solide, préalablement enrobée dans une résine, sur une grille en cuivre recouverte par un film de carbone
2) Les images de microscopie électronique en transmission en haute résolution ont été réalisées au « Fritz-Haber-Institute of the Max Planck Society of Berlin », Allemagne, sur un microscope électronique en transmission, Philips CM200. La tension d'accélération était de 200 kV.

### Diffraction des rayons X sur poudre aux grands angles (WAXS) :

La diffraction des rayons X aux grands angles a été effectuée au CEMES à Toulouse sur un appareil SEIFERT XRD en balayant une gamme d'angles de 0° < 2θ < 65°. Du signal diffracté, est extraite une fonction appelée « intensité réduite », ensuite la transformée de fourrier de celle-ci permet l'obtention de la taille des cristallites de platine (en utilisant un modèle cubique face centré).

### Diffraction des rayons X sur poudre aux petits angles (XRD) :

Cette analyse a été effectuée avec un appareil de type Bruker D8 Advance diffractometer (33 kV & 45 mA) en utilisant une anode de Cuivre (CuKα, λ = 0,154 nm) au "Centre de diffractométrie H. Longchambon", UCBL, Lyon, France. Les diffractogrammes ont été collectés sur une gamme d'angles 2θ de [0,5°-10,0°] avec un balayage de 0,1°/min. Les distances inter-réticulaires d(hkl) ont été calculés en utilisant la loi de Bragg's (nλ = 2dsinθ). Le paramètre de maille (a₀) pour un matériau mésoporeux de structure hexagonale 2D est donné par la relation a₀ = 2d (100)/ √3.

### Spectroscopie de Rayons X photo-électronique :

Cette analyse a été effectuée avec un spectromètre AXIS ULTRA DLD de KRATOS ANALYTlCAL, utilisant une source d'Aluminium monochromatisé d'une énergie de 20eV et un système de neutralisation des charges coaxiales. Le vide dans la chambre d'analyse était supérieur à 5.10⁻⁸ Pa. Les spectres représentant les niveaux d'énergie du Pt4f, du Si2p et d'O1s ont été mesurés à un angle normal par rapport au plan de la surface. Les effets de charges des spectres haute résolution ont été corrigés en prenant comme référence la valeur de 284.5 eV pour le pic du niveau C1s. La fonction utilisée pour les décompositions des pics, est une composition de fonctions Gaussienne et Lorentzienne, avec 40 % de Lorentzien (p), et après soustraction d'un fond d'électrons secondaires de type Shirley.

### Mesures d'adsorption et désorption d'azote.

Les mesures d'adsorption et désorption d'azote ont été effectuées à - 196°C (77K) en utilisant un appareil Micromeritics ASAP 2020. Avant analyse, les échantillons ont été dégazés à 10⁻⁴ Pa à 350°C (623K) pendant 2 h. La distribution des diamètres des pores ainsi que la taille moyenne des pores (dp) ont été calculés en utilisant la méthode BJH (Barrett-Joyner-Halenda). Les surfaces spécifiques (S_{BET}) ont été calculées avec l'équation BET (Brunauer-Emmett-Teller). Les épaisseurs des murs (ep) des solides ont été calculés en utilisant la formule suivante: ep = √3a₀/2 - dp.

### Mesures d'adsorption H₂ et O₂ :

Les mesures d'adsorption d'H₂ ont été effectuées à 25°C (298 K) dans un système conventionnel en Pyrex pour la volumétrie d'adsorption. Le vide de 10⁻⁴ Pa (10⁻⁶ mbar) est atteint avec une pompe à diffusion de mercure. Les pressions d'équilibre ont été mesurées avec une gauge Texas Instrument (gamme de pression entre 0- 100 kPa (1000 mbar) avec une précision de 0,01 kPa (0,1 mbar)). L'échantillon à analyser est placé dans une cellule en Pyrex et dégazé à 25°C (298 K) puis à 300°C (573 K) sous pression réduite pendant 3 h avant les mesures de chimisorption. Les rapports H₂/Pt ont été calculés en extrapolant à pression nulle, l'isotherme d'adsorption obtenu. La dispersion des nanoparticles de platine, défini comme le rapport entre le nombre d'atomes de platine de surface et le nombre d'atomes de platine totale (Pt_{surface}/Pt,) a été déduit en considérant une stoechiométrie de 1,0 H/Pt_{surface} et 1,0 O/P_{surface}.

### Chromatographie en phase gaz :

Les analyses ont été effectuées sur un appareil Hewlett Packard 5890 séries II gas chromatography (GC) équipé d'un détecteur à ionisation de flamme (FID) et d'une colonne de type KCl/Al₂O₃ (50 m x 0,32 mm).

### A. Synthèse de ligands silylés donnant un caractère hydrophile aux particules :

### I. Synthèse du 3-chloropropylsilane (C₃H₉ClSi) :

Le 3-chloropropyltriéthoxysilane (50 mmoles) est introduit sous argon goutte à goutte à 0°C (273 K) dans une solution éthérée (50 ml) de LiAlH₄ (50 mmoles). Le mélange est lentement porté à température ambiante puis agité pendant 12 heures. Ensuite le LiAlH₄ n'ayant,pas réagi est détruit avec 10 mL d'acétate d'éthyle. La suspension est alors filtrée. Le diéthyléther est ensuite évaporé sous pression réduite de 0,1 Pa (10⁻³ mbar). Le résidu est distillé sous argon (Téb = 95°C (368 K)). Le produit est obtenu sous la forme d'un liquide incolore.
¹H NMR (300 MHz, CD₂Cl₂): 0.76; 1.74 ; 3.3 ; 3.54 ppm
¹³C NMR (75 MHz, CD₂Cl₂): 10.5 ; 12.2 ; 27.5 ppm
²⁹Si NMR (75 MHz, CD₂Cl₂) : -58.7 ppm
Infra-Rouge : ν (Si-H) : 2150 cm⁻¹, ν (C-H aliphatique) : 3000-2900 cm⁻¹

### Il. Synthèse du N-(3-propyltrihydrogenosilyl)-imadazole (C₆H₁₂N₂Si) :

Le N-(3-propyltriethoxysilyl)-imidazole (50 mmoles) est introduit sous argon goutte à goutte à 0°C (273 K) dans une solution éthérée (50 ml) de LiAlH₄ (50 mmoles). Le mélange est lentement porté à température ambiante puis agité pendant 12 heures. Ensuite le LiAlH₄ n'ayant pas réagi est détruit avec 10 mL d'acétate d'éthyle. La suspension est, alors, filtrée. Le diéthyléther est ensuite évaporé sous pression réduite de 0,1 Pa (10⁻³ mbar). Le produit est obtenu sous la forme d'une huile incolore.
¹H NMR (300 MHz, CD₂Cl₂): 1.3-1.5 ; 2.55 ; 3.54 ; 7 ppm
²⁹Si NMR (75 MHz, CD₂Cl₂): -58.7 ppm

### III. Synthèse du Chlorobenzylsilane (C₇H₉ClSi):

Le chlorobenzyltriéthoxysilane (50 mmoles) est introduit au goutte à goutte sous argon à 0°C (273 K) dans une solution éthérée (50 ml) de LiAlH₄ (50 mmoles). Le mélange est lentement porté à température ambiante puis agité pendant 12 heures. Ensuite le LiAlH₄ n'ayant pas réagi est détruit avec 10 mL d'acétate d'éthyle. La suspension est alors filtrée. Le diéthyléther est ensuite évaporé sous pression réduite de 0,1 Pa (10⁻³ mbar). Le produit est obtenu sous la forme d'un liquide incolore.
¹H NMR (300 MHz, CD₂Cl₂): 0.76 ; 1.74 ; 3.3 ; 3.54 ppm
¹³C NMR (75 MHz, CD₂Cl₂): 10.5 ; 12.2 ; 27.5 ppm
Infra-Rouge : ν (Si-H) : 2140cm⁻¹, ν (C-H_{aliphatique et aromatique}) : 3100-2900 cm⁻¹ ; 700-900 cm⁻¹

### IV. Chlorodiméthylsilane (C₂H₇ClSi) :

Produit commercial (ABCR), utilisé tel quel.

### B. Synthèse de suspensions colloïdales de nanoparticules hydrophiles :

### I. Exemple d'une solution colloïdale hydrophile de Pt stabilisée par le 3-chloropropylsilane :

100 mg (0,15 mmol) de Pt (dba)₂ (avec dba = dibenzylideneacétone) sont placés dans un réacteur en verre et tirés sous pression réduite pendant 30 minutes à température ambiante. 90 ml de THF (TétraHydroFurane) sont ensuite ajoutés. 10 ml de THF contenant 25 mg de 3-chloropropylsilane (0,15 mmol) sont ajoutés à température ambiante. La solution obtenue est pressurisée à 300 kPa (3 bars) d'hydrogène sous agitation pendant 12 heures.

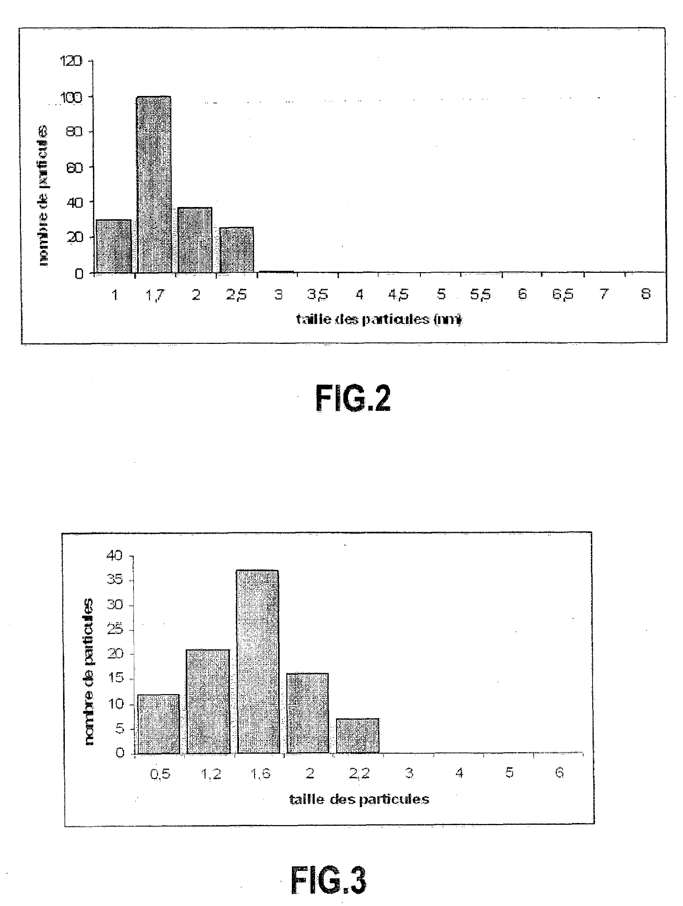

La **Figure 2** représente l'histogramme de la taille des particules obtenues.

Un test d'hydrophilie est réalisé en plaçant la suspension de particules obtenue dans un récipient contenant un mélange biphasique eau/heptane, l'eau étant située en dessous de l'heptane dans le récipient : les particules métalliques vont dans la phase aqueuse et non dans la phase heptane, ce qui met en évidence leur caractère hydrophile.

### II. Exemple d'une solution colloïdale hydrophile de Ru stabilisée par le 3-chloropropylsilane :

100 mg (0,29 mmol) de Ru (COD)(COT) (avec COD = cyclooctadiène et COT = cyclooctatétraène) sont placés dans un réacteur en verre et tirés sous pression réduite pendant 30 minutes à température ambiante. 90 ml de THF sont ensuite ajoutés. 10 ml de THF contenant 40 mg de 3-chloropropylsilane (0,29 mmol) sont ajoutés à température ambiante. La solution obtenue est pressurisée à 300 kPa (3 bars) d'hydrogène sous agitation pendant 12 heures.

La **Figure 3** représente l'histogramme de la taille des particules obtenues.

Un test d'hydrophilie est réalisé comme précédemment et met en évidence le caractère hydrophile des particules obtenues.

### III. Exemple d'une solution colloïdale hydrophile de Pt stabilisée par le chlorobenzylsilane :

100 mg (0,15 mmol) de Pt (dba)₂ sont placés dans un réacteur en verre et tirés sous pression réduite pendant 30 minutes à température ambiante. 90 ml de THF sont ensuite ajoutés. 10 ml de THF contenant 23 mg (0,15 mmol) de chlorobenzylsilane sont ajoutés à température ambiante. La solution obtenue est pressurisée à 300 kPa (3 bars) d'hydrogène sous agitation pendant une nuit.

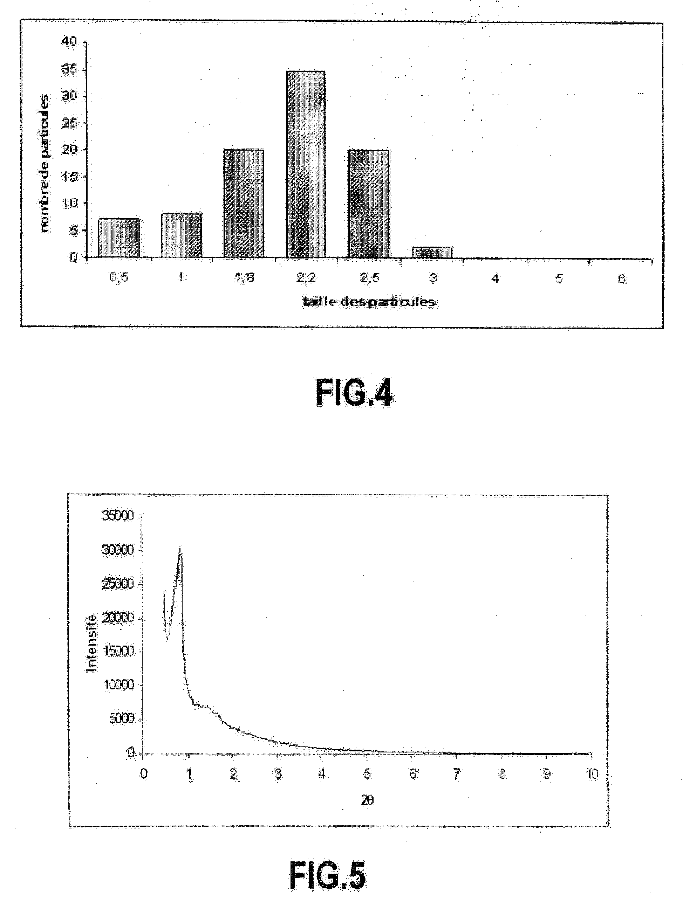

La **Figure 4** représente l'histogramme de la taille des particules obtenues.

Un test d'hydrophilie est réalisé comme précédemment et met en évidence le caractère hydrophile des particules obtenues.

### IV. Exemple d'une solution colloïdale hydrophile de Pt stabilisée par le chlorodlméthylsilane :

100 mg (0,15 mmol) de Pt (dba)₂ sont placés dans un réacteur en verre et tirés sous pression réduite pendant 30 minutes à température ambiante. 90 ml de THF sont ensuite ajoutés. 10 ml de THF contenant 15 mg (0,15 mmol) de chlorodiméthylsilane sont ajoutés à température ambiante. La solution obtenue est pressurisée à 300 kPa (3 bars) d'hydrogène sous agitation pendant une nuit.

Un test d'hydrophilie est réalisé comme précédemment et met en évidence le caractère hydrophile des particules obtenues.

### V. Exemple d'une solution colloïdale hydrophobe de Pt stabilisée par l'octylsilane :

100 mg (0,15 mmol) de Pt (dba)₂ sont placés dans un réacteur en verre et tirés sous pression réduite pendant 30 minutes à température ambiante. 90 ml de THF sont ensuite ajoutés. 10 ml de THF contenant 20 mg (0,15 mmol) d'octylsilane sont ajoutés à température ambiante. La solution obtenue est pressurisée à 300 kPa (3 bars) d'hydrogène sous agitation pendant une nuit.

Un test d'hydrophilie est réalisé comme précédemment et met en évidence le caractère hydrophobe des particules obtenues.

### C. Matériaux contenant les nanoparticules métalliques stabilisées par ligands silanes dans les murs :

### I. Silice contenant les nanoparticules de Pt stabilisées par des ligands 3-chloropropylsilane dans les murs :

### 1. Synthèse :

Dans un Erlenmeyer de 150 mL, 0,5g (86 µmol) du tensioactif structurant P123 (Pluronic 123 (Aldrich 98%): H-(O-CH₂-CH₂-)₂₀-(O-CH₂(CH₃)-CH₂)₇₀-(O-CH₂-CH₂)₂₀-OH) est ajouté à 50 mL d'eau distillée contenant 20 mg de NaF, sous agitation vigoureuse. Après obtention d'une solution homogène, 20 ml d'une solution colloïdale de nanoparticules de Platine hydrophiles (24 µmol) préparées précédemment au paragraphe B-I en solvant THF sont ajoutés. Le mélange est agité vigoureusement pendant 2 heures. Le THF est ensuite évaporé sous pression réduite. Dans un deuxième erlenmeyer, 5g (24mmol) de TEOS (TetraEthylOrthoSilicate) sont mélangés à une solution aqueuse d'HCl (pH final =1,5) pendant 3 h. Les deux mélanges réactionnels sont portés à 35°C puis mis en contact. Le mélange réactionnel est finalement agité à 35°C pendant 24h. La suspension est ensuite filtrée et le solide obtenu, lavé avec deux fois 20mL d'eau, d'éthanol, d'acétone et d'éther.

### 2. Caractérisation avant traitement :

La **Figure 5** présente le diffractogramme sur poudre des Rayons X aux petits angles du solide obtenu et met en évidence la structuration du matériau obtenu.

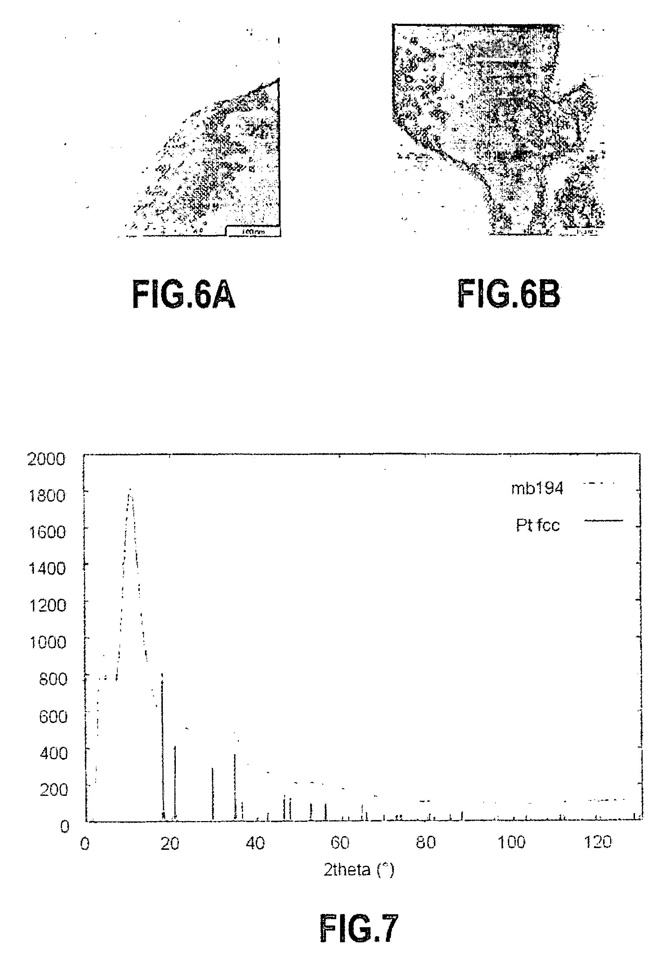

Les **Figures 6A** et **6B** sont des images par microscopie électronique en transmission montrant la structure de type Hexagonale 2D des canaux poreux et la présence de particules de Pt au sein du matériau obtenu.

La **Figure 7** présente le spectre WAXS du matériau contenant des particules de Pt dans les murs avant élimination du tensioactif et des chaînes carbonées des ligands stabilisants (courbe MB194) ainsi que le spectre simulé d'une cristallite de Pt de 2 nm en la modélisant avec un empilement atomique de type cubique face centré (courbe Pt fcc). La taille des particules données par WAXS avant traitement était de 2 nm et la courbe Pt fcc est une simulation correspondant à de telles particules. Il apparaît donc qu'au sein du matériau, les particules sont restées de petites tailles et présentent une taille comparable à celle qu'elles avaient en suspension colloïdale.

### 3. Caractérisation après traitement :

### a) Traitement par calcination à 350°C (623K) :

La calcination consiste à introduire 1 g de matériau non extrait placé dans un réacteur sous flux d'air sec. Le réacteur est ensuite chauffé jusqu'à 623K avec une rampe de température de 2 K par minute.

### Caractérisation du matériau après traitement :

### Caractéristiques structurales du matériau :

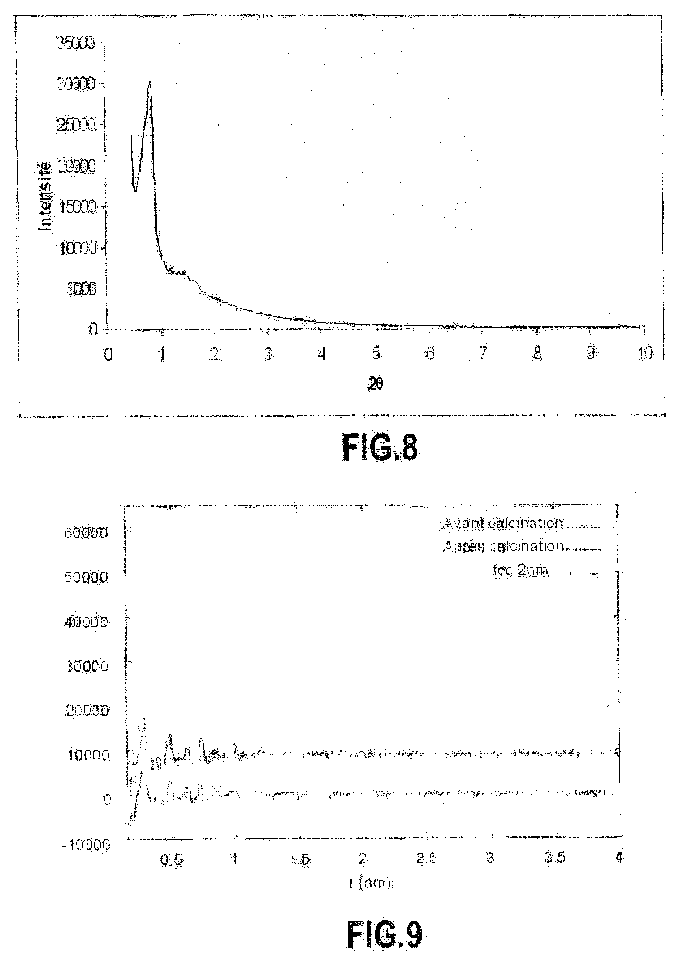

La **Figure 8** donne le diffractogramme sur poudre de RX aux petits angles (a = 115 Å) du matériau obtenu. Ce diffractogramme présente un pic de diffraction attribuable à la diffraction des familles de plans réticulaires (1,0,0), et deux harmoniques correspondant à la diffraction des plans (1,1,0) et (2,0,0). Le matériau possède donc une structuration poreuse sous forme d'un réseau hexagonal 2D. Ceci est confirmé par microscopie électronique en transmission.

La **Figure 9** présente en trait plein, les transformées de fourrier expérimentales (avant calcination _ courbe du dessus superposé au modèle _ et après calcination _ courbe du dessous) et en pointillés, les modèles théoriques d'une cristallite de Pt de 2 nm en réseau cristallographique de type cubique face centrée (fcc). Là encore, il apparaît qu'après traitement, au sein du matériau, les particules sont restées de petites tailles et présentent une taille comparable à celle qu'elles avaient en suspension colloïdale.

Caractéristiques texturales du matériau :
S_{BET} : 958±20m²/g
Volume poreux : 1,0±0.1m³/g
Diamètre des pores: 6±1 nm
Taille des murs : 8±1nm

Caractéristiques structurales du matériau et présence de nanoparticules :

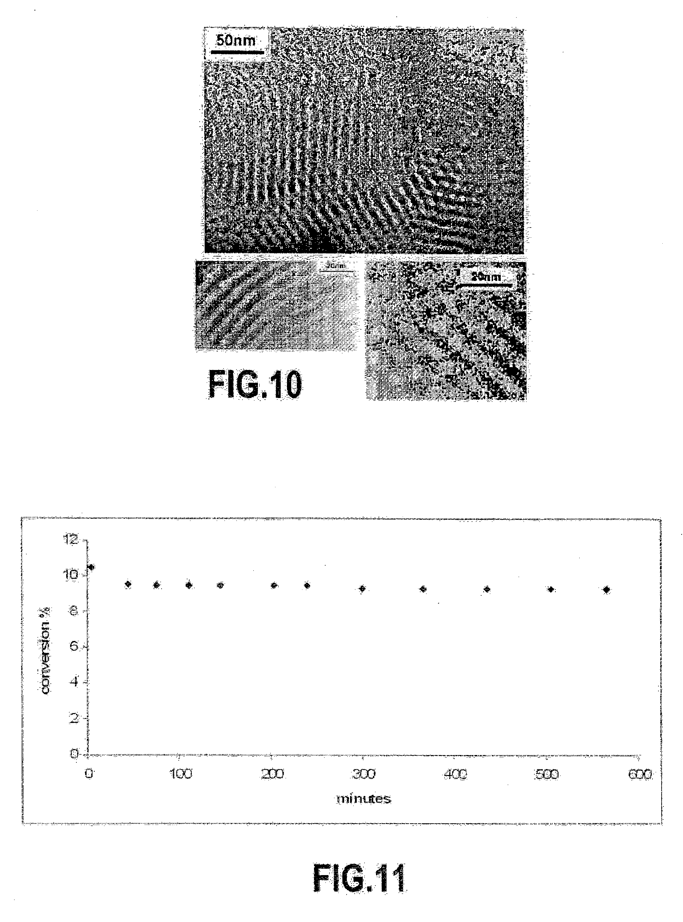

La **Figure 10** présente différentes images par microscopie électronique en transmission après calcination du matériau, qui mettent en évidence la présence de particules métalliques au sein des murs de la charpente du matériau.

### Performances catalytiques :

### Hydrogénation du propène en réacteur à flux continu

Le catalyseur (7 mg, 0,107 µmol de Pt) dilué dans du carbure de silicium (50mg) est chargé dans un réacteur en verre. Celui-ci est parcouru par un gaz inerte (l'hélium) pendant une heure. Ensuite le Pt est réduit sous H₂ pendant 3h à 573K. Enfin, le réacteur est mis en contact avec le mélange réactionnel propene/H₂/He (20/16/1,09) cm³/min. La pression est de 100kPa (1 bar). La réaction est suivie par chromatographie en phase gazeuse.

Les résultats sont présentés **Figure 11** qui montre le taux de conversion du propène en fonction du temps.

Performances expérimentales obtenues :
Fréquence de tours (TOF) (à 10 minutes) = 180 min⁻¹

### b) Après traitement Fer/UV :

**Protocole :** Extraction du matériau contenant des particules de Platine stabilisées par les ligands 3-chloropropylsilanes par le traitement Fer/UV

Le matériau (100mg) est mis en suspension dans un bêcher de 200 mL contenant 50 mL d'une solution aqueuse d'acide sulfurique à pH=3. Ensuite 10 mg (0,36 mmol) de FeSO₄ sont ajoutés. Le mélange est agité vigoureusement à température ambiante et à l'air sous irradiation UV, avec une puissance d'environ 5watts/m² (lampe à vapeur de mercure, Philips HPK 125W) pendant 5h. Le solide est alors filtré puis lavé avec 20 mL d'eau distillée et d'acétone. Il est ensuite séché sous pression réduite 10⁻³Pa (10⁻⁵ mbar) à 135°C pendant 12 h.

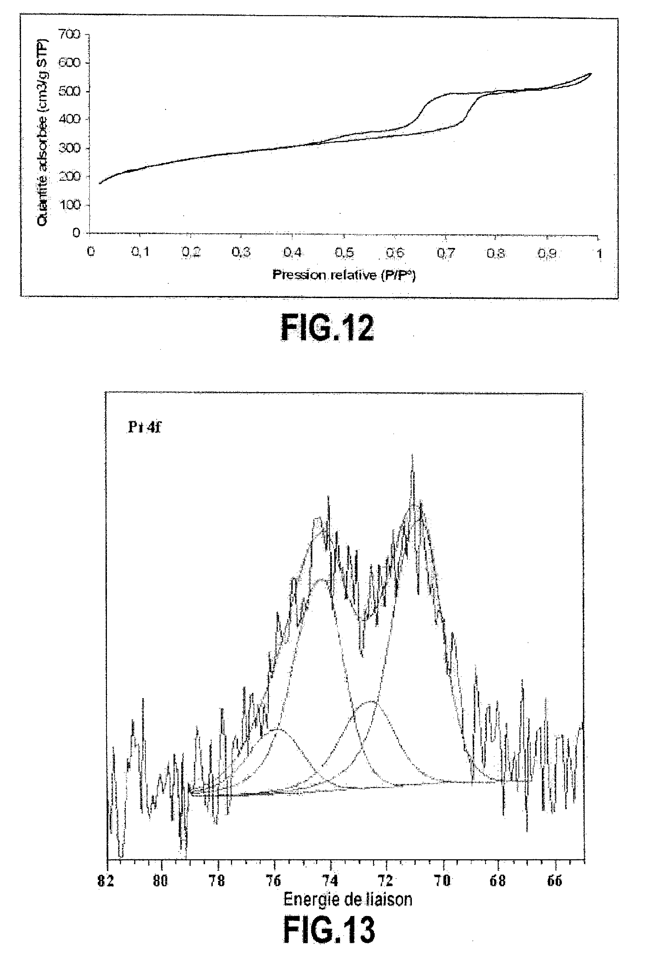

La **Figure 12** présente l'isotherme d'adsorption/désorption d'azote à -196°(77K) du matériau contenant les particules de Platine obtenu après traitement. Le maténau contenant des nanoparticules de métal 0 dans les murs présente un isotherme caractéristique de solides mesoporeux (isotherme de type IV) et dont la distribution de la population poreuse est étroite.

La **Figure 13** présente le spectre XPS du matériau contenant des particules de Pt dans les murs du matériau.
Energies de liaison : (eV)
Réf interne Si 2p = 103.6 eV

| | **Pt 4f_{7/2}** | **Si 2p** |
|---|---|---|
| Matériau contenant des Particules hydrophiles dans les murs après calcination | 71,1 (75 %) | 103,6 |
| | 72,6 (25 %) | |
| Particules de Pt nues supportés sur Al₂O₃ | 71,5 (100 %) | 103,6 |

### Decomposition du Pt 4f

un doublet Pt 4f_{7/2} - Pt 4f_{5/2} (71.0 eV - 74.25 eV) attribué à Pt-Pt

un doublet Pt 4f_{7/2} - Pt 4f_{5/2} (72.5 eV - 75.75 eV) attribué à Pt-Si

Les Pt-Pt° représentent environ 75% de la quantité total d'atomes de Pt. La présence de liaison Si-Pt_{surface} est détecté (25%) et même après calcination à 623K aucun Pt oxydé n'a été détecté.

On observe la présence d'atomes de silicium liés aux atomes Pt de surface avant et après traitement thermique, ce qui confirme le caractère non-échangeable des ligands de surface liés aux particules. En outre, l'absence d'oxyde de platine confirme bien la nature purement métallique (métal 0) des particules incorporées dans le matériau.

### II. Silice contenant les nanoparticules de Ru stabilisées par des ligands 3-chloropropylsilane dans les murs :

### 1. Synthèse :

Dans un Erlenmeyer de 150 mL, 0,5g (86 µmol) du tensioactif structurant P123 est ajouté à 50 mL d'eau distillée contenant 20 mg de NaF, sous agitation vigoureuse. Après obtention d'une solution homogène, 20 ml d'une solution colloïdale de nanoparticules de Ru hydrophiles (24 µmol) préparées précédemment au paragraphe B-I en solvant THF sont ajoutés. Le mélange est agité vigoureusement pendant 2 heures. Le THF est ensuite évaporé sous pression réduite. Dans un deuxième Erlenmeyer, 5g (24 mmol) de TEOS sont additionnés à une solution aqueuse d'HCl (pH final =1,5) et hydrolysés pendant 3 h. Les deux mélanges réactionnels sont portés à 35°C. Les deux mélanges sont ensuite mis en contact et le tout est finalement agité à 35°C pendant 24h. Le solide gris-beige obtenu est filtré puis lavé avec deux fois 20mL d'eau, d'éthanol, d'acétone et d'éther.

### 2. Caractérisation avant traitement :

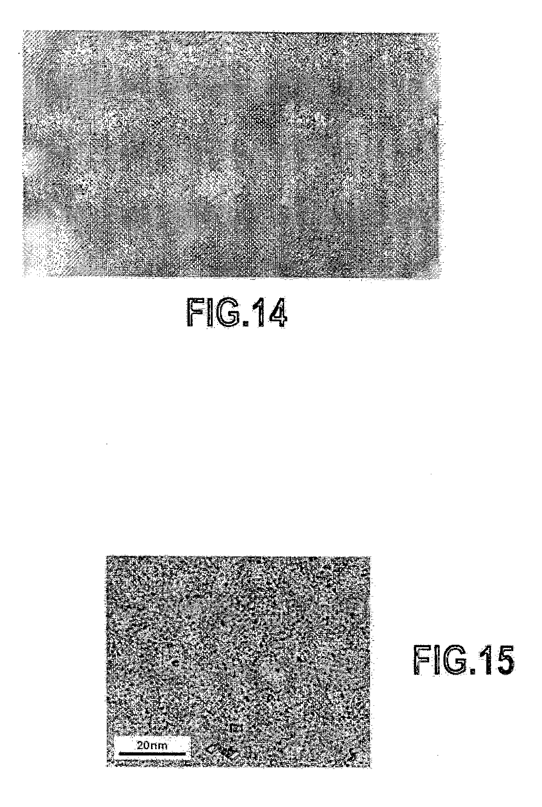

La **Figure 14** présente une image par microscopie électronique en transmission, qui met bien en évidence la structuration du matériau, de type hexagonal 2D.

### 3. Caractérisation après calcination à 350°C (623K) (conformément au paragraphe C.I.3.a) :

Le matériau contenant des nanoparticules de ruthénium 0 dans les murs présente un isotherme caractéristique d'un solide mésoporeux (isotherme type IV) et une distribution de la population mésoporeuse étroite.

Texture du matériau :
S_{BET} : 960±20m²/g
S_{mésopores} : 630±20m²/g
S_{micropores} : 340±20m²/g
Volume poreux :1,2 ±0,1 m³/g
Diamètre des pores: 6±1 nm
Taille des murs : 8±1nm

### III. Silice contenant les nanoparticules de Pt stabilisées par des ligands Chlorobenzylsilane dans les murs :

### 1. Synthèse :

Dans un Erlenmeyer de 150 mL, 0,5g (86 µmol) du tensioactif structurant P123 sont ajoutés à 50 mL d'eau distillée contenant 20 mg de NaF, sous agitation vigoureuse. Après obtention d'une solution homogène, 20 ml d'une solution colloïdale de nanoparticules de Platine hydrophiles (24 µmol) préparées précédemment au paragraphe B-III en solvant THF sont ajoutés. Le mélange est agité vigoureusement pendant 2 heures. Le THF est ensuite totalement évaporé sous pression réduite. Dans un deuxième Erlenmeyer 5g (24mmol) de TEOS sont additionnés à une solution aqueuse d'HCL (pH final =1,5) et hydrolysés pendant 3 h. Les deux mélanges réactionnels sont portés à 35°C. Les deux mélanges sont ensuite mis en contact et le tout est finalement agité à 35°C pendant 24h. Le solide gris-beige obtenu est filtré puis lavé avec deux fois 20mL d'eau, d'éthanol, d'acétone et d'éther.

### 2. Caractérisation avant traitement :

La **Figure 15** présente une image par microscopie électronique en transmission, qui met bien en évidence la structuration du matériau, de type hexagonal 2D et la présence de particules dans les murs.

### IV. Silice contenant les nanoparticules de Pt stabilisées par des ligands Chloropropylsilanes dans les murs et des particules de Pt stabilisées par des ligands octylsilane dans les pores :

### 1. Synthèse :

Dans un Erlenmeyer de 150 mL, 0,5g (86 µmol) du tensioactif structurant P123 sont ajoutés à 50 mL d'eau distillée contenant 20 mg de NaF, sous agitation vigoureuse. Après obtention d'une solution homogène, 20 ml d'une solution colloïdale de nanoparticules de Platine hydrophiles (24 µmol) préparées au paragraphe B-I en solvant THF sont ajoutés. Ensuite 30 mL d'une solution colloïdale de nanoparticules de Platine hydrophobes préparées au paragraphe B-V (0,045 mmol) en solvant THF sont également ajoutés Le mélange est agité vigoureusement pendant 2 heures. Le THF est ensuite totalement évaporé sous pression réduite. Dans un deuxième Erlenmeyer, 5g (24mmol) de TEOS sont mélangés à une solution aqueuse d'HCl (pH final =1,5) pendant 3 h. Les deux mélanges réactionnels sont ensuite portés à 35°C puis mis en contact. La solution obtenue est agitée à 35°C pendant 24h. La suspension obtenue est ensuite filtrée et le solide obtenu, avec deux fois 20mL d'eau, d'éthanol, d'acétone et d'éther.

### 2. Caractérisation après calcination à 350 ° C (623K) (conformément au paragraphe C.I.3a)

D'après les mesures d'adsorption/désorption d'azote, le matériau présente les caractéristiques suivantes, à savoir une porosité de type méso/microporeuse avec un grand volume poreux et une population mésoporeuse centrée sur 6 nm et une épaisseur de murs de 7 nm :
S_{BET} : 870±20m²/g
S_{mésopores} : 540±20m²/g
S_{micropores} : 330±20m²/g
Volume poreux :1,0 ±0,1 m³/g
Diamètre des pores: 6±1 nm
Taille des murs : 7±1nm

### V. Oxyde mixte SiO₂/TiO₂ contenant les nanoparticules de Pt stabilisées par des ligands 3-chloropropylsilane dans les murs :

Dans un Erlenmeyer de 150 mL, 0,5g (86 µmol) du tensioactif structurant P123 est ajouté à 50 mL d'eau distillée contenant 20 mg de NaF, sous agitation vigoureuse. Après obtention d'une solution homogène, 20 ml d'une solution colloïdale de nanoparticules de Platine hydrophiles (24 µmol) en solvant THF sont ajoutés. Le mélange est agité vigoureusement pendant 2 heures. Le THF est ensuite totalement évaporé sous vide (10⁻³ mbar). Dans un deuxième Erlenmeyer, 4,86g (23mmol) de Tetraméthoxysilane sont mélangés à une solution aqueuse d'HCl (pH final =1,5) pendant 2 h. Ensuite 20 mg (0,6mol) de tétraisopropoxysilane de titane sont additionnés, le tout est agité 20 minutes. Finalement les deux mélanges réactionnels sont portés à 35°C puis mis en contact et agité à cette température pendant 24 h. La suspension est ensuite est filtrée et le solide obtenu est lavé avec deux fois 20mL d'eau, d'éthanol, d'acétone et d'éther.

### Caractérisation après calcination à 350 ° C (623K)

D'après les mesures d'adsorption/désorption d'azote, le matériau présente les caractéristiques suivantes
S_{BET} : 500±20m²/g
S_{mésopores} : 360±20m²/g
S_{micropores} : 140±20m²/g
Volume poreux :0,6 ±0,1 m³/g
Diamètre des pores: 6±1 nm

### D. Comparaison avec d'autres matériaux contenant des particules dans les pores ou en surface :

### Silice contenant les nanoparticules de Pt stabilisées par des ligands octylsilane dans les pores :

Dans un Erlenmeyer de 150 mL, 1,7 g (0,29 mmol) du tensioactif P123 sont ajoutés à une solution d'acide chlorhydrique à pH = 1,5 (63 mL) sous agitation vigoureuse. Après obtention d'une solution homogène, 30 ml d'une solution colloïdale de nanoparticules de Platine (0,045 mmoles) dans le tetrahydrofurane (Pelzer, K.; et al. Chem. Mater. 2004, 16, 4937-4941). sont ajoutés. Le mélange est agité vigoureusement pendant 2 heures. 3,53 g (17mmol) de TEOS sont ensuite rapidement additionnés et le mélange est laissé sous agitation pendant 3h. Le mélange réactionnel est alors porté à 45°C puis 25 mg de NaF sont rapidement additionnés. Le mélange est finalement agité à 45°C pendant 48h. Le solide blanc obtenu est filtré puis lavé avec deux fois 20mL, d'eau, d'éthanol, d'acétone et d'éther.

Le matériau obtenu est traité par calcination à 350°C.

Les diffractogrammes des RX sur poudre aux petits angles, avant et après calcination, sont identiques et montrent un pic correspondant à 2θ = **0,9°**.

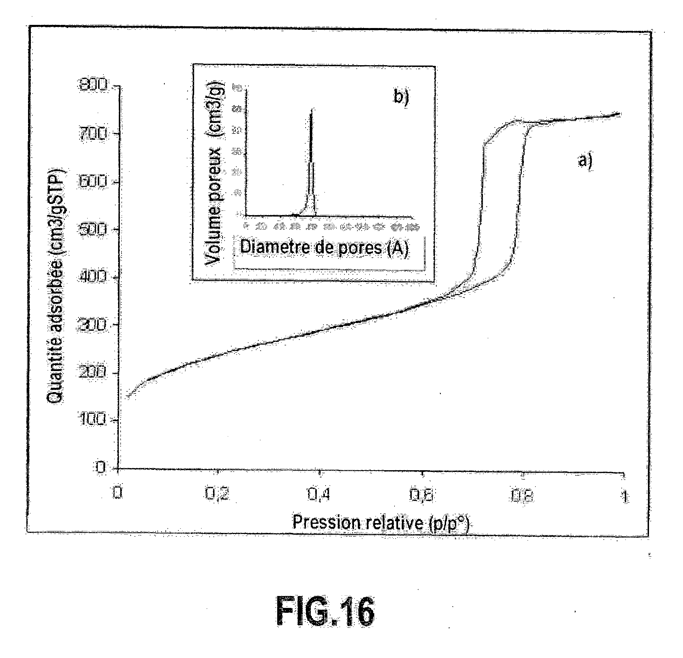

Les **Figures 16 a)** et **b)** présentent respectivement l'isotherme d'adsorption/désorption d'azote de type IV à -196°C (77K) du matériau obtenu et la distribution poreuse. D'après les mesures d'adsorption/désorption d'azote le matériau présente les caractéristiques suivantes, à savoir une porosité de type majoritairement mésoporeuse avec une population poreuse centrée sur 8,5 nm et une épaisseur de murs de 3 nm :
S_{BET} : 1050±20m²/g
S_{mésopores} : 840±20m²/g
S_{micropores} : 230±20m²/g
Volume poreux : 1,5 ±0,1 m³/g
Diamètre des pores: 8,5±1 nm
Taille des murs : 3±1nm

L'étude par WAXS montre que la taille des particules passe de 2 à 4 nm pour le matériau contenant le Pt dans les pores, alors les particules restent à 2 nm pour le matériau contenant les particules dans les murs.

### 3. Comparaison des activités catalytiques

### Hydrogénation du propène en flux continu

7 mg de catalyseur (0,3%wt de Pt ,0.107 µmol of Pt) sont chargés dans un réacteur à lit fixe sous flux continu. Le réacteur est purge avec de l'Helium pendant 1h. Le catalyseur est placé ensuite sous courant d'H₂ pendant 3h à 573 K avant d'être connecté à un mélange propene/H₂/He à une pression de 100 kPa (1 bar). La réaction est suivie par chromatographie gazeuse.

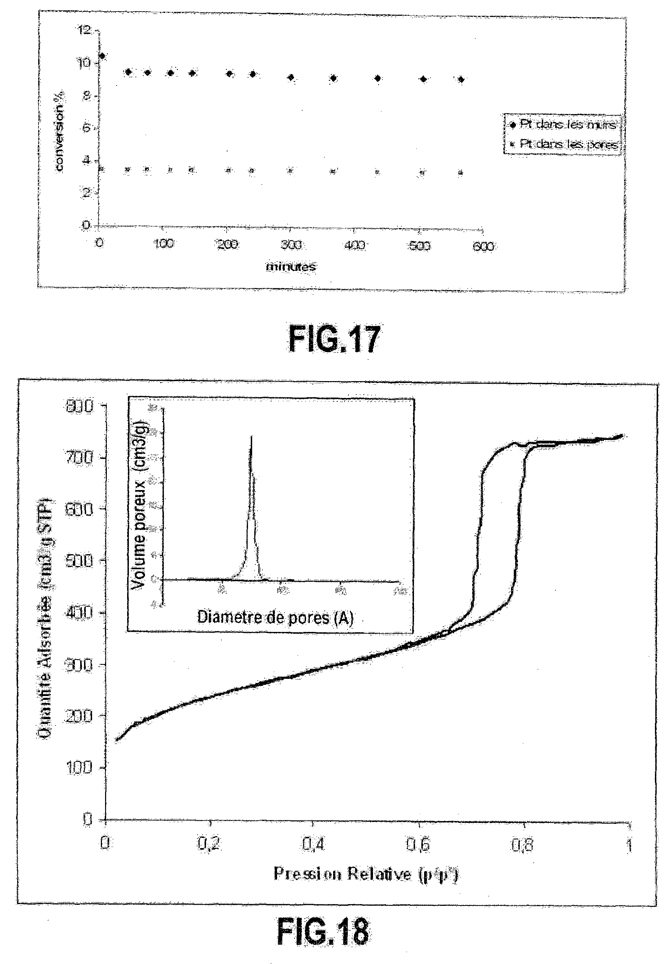

La **Figure 17** compare les taux de conversion obtenus par hydrogénation du propène en réacteur dynamique, avec le matériau selon l'invention, tel que décrit au paragraphe C.3.1 et celui obtenu précédemment avec des particules de Pt dans les pores.

### Performances expérimentales obtenues :

Fréquence de tours (TOF) (à 10 minutes) pour le matériau contant les particules de Pt dans les pores= 55 min⁻¹

Fréquence de tours (TOF) (à 10 minutes) pour le matériau contant les particules de Pt dans les murs= 180min⁻¹

En conclusion, les activités catalytiques obtenues sont comparables, avec même une activité légèrement améliorée dans le cas des matériaux selon l'invention.

Par conséquent, les particules localisées dans les murs restent accessibles, et donc actives et réactives.

### Silice contenant les nanoparticules de Pt stabilisées par des ligands octylsilane préparées selon le paragraphe B.V dans les pores et obtenu par imprégnation d'une solution colloïdale sur support mésostructuré de type SBA-15 :

### Synthèse (Zhao, D. et al. Science 1998, 279, 548-552) :

### 1^{ère} étape (synthèse du support mésostructuré) :

Dans un Erlenmeyer de 150 mL, 1,7 g (0,29 mmol) de P123 sont ajoutés à une solution d'acide chlorhydrique à pH = 1,5 (63 mL) sous agitation vigoureuse. Après obtention d'une solution homogène, le TEOS est additionnés et le mélange réactionnel est laissé sous agitation pendant 3h. La solution est alors portée à 45°C puis 25 mg de NaF sont rapidement additionnés. Le mélange est finalement agité à 45°C pendant 48h. Le solide blanc obtenu est filtré puis lavé avec deux fois 20mL, d'eau, d'éthanol, d'acétone et d'éther.

La **Figure 18** présente l'isotherme d'adsorption/désorption d'azote de type IV à -196°C (77K) du matériau obtenu et la distribution poreuse. D'après les mesures d'adsorption/désorption d'azote le matériau présente les caractéristiques suivantes
S_{BET} = 1000 m²/g ; Vp = 1,5 cm³/g ; Dp (adsorption) : 9 nm

### 2ème étape :

Une solution colloïdale de Pt stabilisé par des ligands octylsilanes dans le THF est mise en contact avec le support préalablement mis en suspension dans quelques millilitres de THF. La suspension obtenue est laissée sous agitation pendant 24 h puis le THF est évaporé sous pression réduite 0,1 Pa (10⁻³ mbar). 2g d'une poudre grise est obtenue.

### Caractérisation du matériau après calcination à 350° C (623K)

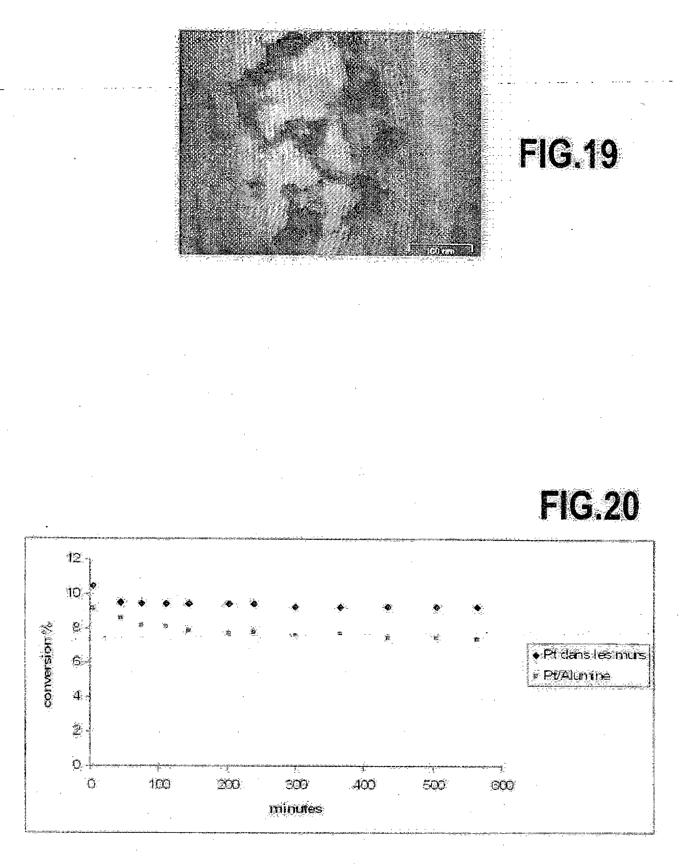

La Figure 19 présente une image par microscopie électronique en transmission du matériau imprégné obtenu après calcination

Il apparait un frittage important des particules au sein du support mésostructuré, ce qui explique une activité catalytique nulle du matériau, après traitement, constatée dans la réaction d'hydrogénation du propène

### Nanoparticules de Pt déposées sur alumine (catalyseur industriel monométallique chargé en Pt (0,32 % poids) sur alumine gamma à 200 m²/g, fourni par Axens Group Technologies)

### 1. Caractéristiques :

Tailles des particules = 1 nm (dispersion = 80 à 90%)
% de Pt dans le matériau = 0.3 %wt

### 2. Comparaison des activités catalytiques

La **Figure 20** compare les taux de conversion obtenus pour l'hydrogénation du propène avec le catalyseur de référence et le matériau selon l'invention, tel que décrit au paragraphe C.3.1

### Performances expérimentales

TOF(10 min) matériau de référence = 60min⁻¹

TOF(10 min) matériau contenant les particules de Pt dans les Murs = 180 min⁻¹

Il apparaît donc que les activités sont comparables. Ces résultats prouvent que les particules de Pt restent actives et réactives malgré leur localisation dans les murs d'une charpente poreuse, et présentent une activité comparable à des particules de Pt nues, en surface d'un support alumine.

### Catalyse d'hydrogénation du styrène à température ambiante en réacteur fermé :

### Protocole :

Le catalyseur étudié (0,96 µmol de Pt_{surface}), le styrène (8862 µmol, 9200 equiv.) et le solvant l'heptane (50 mL) sont chargés dans un réacteur batch de 100 mL sous argon. Le mélange réactionnel est agité puis 3500 kPa (35 bars) d'H₂ sont ajoutés. Pendant la réaction de petites quantités du mélange réactionnel sont prélevées et analysées par chromatographie en phase gazeuse afin de suivre la cinétique de la réaction.

### Résultats :

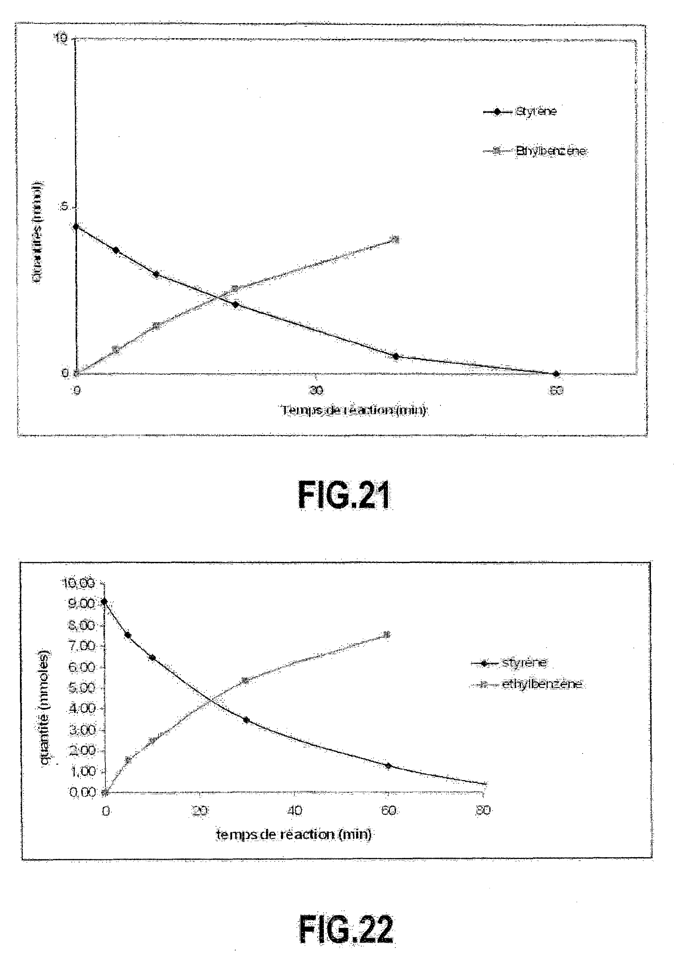

La **Figure 21** présente les quantités de styrène et d'éthylbenzène en fonction du temps, obtenues lors de l'hydrogénation du styrène avec le matériau contenant les particules de Pt dans les murs après calcination, tel que décrit au paragraphe C.3.1 (R=9200 pour 40%dispersion).

La **Figure 22** présente les quantités de styrène et d'éthylbenzène en fonction du temps, lors de l'hydrogénation du styrène avec le catalyseur de référence Pt sur alumine (90% dispersion, particules de Pt d'environ 1 nm, R=5000)

Les activités obtenues sont également comparables :
TOF (10 min) Mat Pt dans Murs = 160 min⁻¹
TOF (10 min)) matériau de référence = 80 min⁻¹

Par conséquent, même dans le cas de réactions catalytiques réalisées sur des molécules plus grosses, telles que le styrène, les particules de Pt localisées dans les murs d'une charpente poreuse restent accessibles et donc actives et réactives.

### E. Comportement de solutions colloïdales de particules hydrophiles stabilisées par des ligands échangeables, en présence d'agent porogène et essai de synthèse d'un matériau organisé contenant des nanoparticules stabilisées par un ligand hydrophile échangeable

Il a été observé que l'utilisation de ligands échangeables pour la stabilisation de nanoparticules conduisait à des échanges de surface avec des molécules présentes lors du procédé sol-gel (agent porogène, alcool issu de l'hydrolyse des précurseurs alcoxysilanes) et que dès lors, l'incorporation de ces nanoparticules dans la charpente silicique était perturbée. Afin de confirmer cela, des nanoparticules de Pt ont été préparées en utilisant un ligand échangeable et hydrophile : le 1,8-octanediol. Après avoir confirmé par le test décrit paragraphe B.I., le caractère hydrophile de ces particules de Pt et vérifié leur petite taille par MET (c.a. 2,4 nm), un aliquot de cette solution (5 mL) a été prélevé et le solvant a été évaporé sous pression réduite. Le résidu contenant les particules a ensuite été solubilisé dans du benzène deutéré (0,4 mL) et la solution ainsi obtenue a été introduite dans un tube RMN. 0,3 équivalent de bromure de cétyl ammonium a ensuite été ajouté et la réaction d'échange a été suivie en RMN ¹H : le signal correspondant au groupement ammonium a progressivement disparu, alors que le signal correspondant au groupement hydroxyle a augmenté (jusqu'à doubler). Ceci confirme donc la réaction d'échange entre un ligand de type ammonium (qui joue également le rôle d'agent porogène) et le ligand hydrophile stabilisant initialement les nanoparticules.

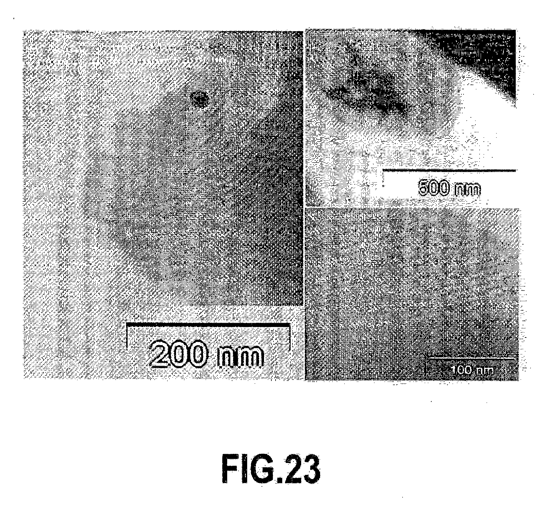

En outre, pour confirmer d'avantage cet échange lors du procédé sol-gel, la synthèse d'un matériau comme décrite au paragraphe C.I.1. a été réalisée à partir de la solution colloïdale précédente. D'après les images de microscopie présentées **Figure 23****,** le matériau obtenu est structuré, néanmoins les nanoparticules ne sont pas incorporées dans les murs de la structure silicique, les nanoparticules se sont agglomérées et ont été rejetées de la structure. Ainsi, comme attendu, un matériau très hétérogène a été obtenu avec des nanoparticules de Pt qui ne sont pas du tout régulièrement distribuées au sein de la matrice et qui, par conséquent, fritteront dès le premier traitement thermique.

## Revendications

1. Procédé de préparation d'un matériau poreux structuré comportant une charpente inorganique structurée composée de murs à base d'oxyde métallique dans lesquels des particules de métal 0 sont incorporées, le dit matériau étant **caractérisé par** la présence d'au moins un pic de diffraction dans un diffractogramme sur poudre des Rayons X aux petits angles associé à une période spatiale de répétition du système structuré qui correspond à la périodicité des pores au sein du matériau,
comprenant les étapes suivantes :
a) disposer d'une suspension de particules hydrophiles de métal 0 stabilisées par des ligands non échangeables qui confèrent leur caractère hydrophile aux particules, dans laquelle le coeur métallique des particules métalliques (hors ligands) est essentiellement sphérique et au moins, pour 50% de la population des particules, le coeur métallique présente un diamètre moyen de 1 à 10 nm, et les particules métalliques étant monodisperses, c'est-à-dire qu'elles présentent une distribution de taille très étroite autour d'une valeur moyenne, de sorte que 50% des particules ont leur taille qui correspond à la taille moyenne ± 0,5 nm,
b) faire croitre la charpente inorganique à partir d'un précurseur inorganique, autour des particules de métal 0 stabilisées par les ligands non échangeables qui confèrent leur caractère hydrophile aux particules, en présence d'un agent porogène, la taille totale des particules hydrophiles stabilisées par les ligands non échangeables qui leur confèrent leur caractère hydrophile étant inférieure ou égale à la taille des murs de la charpente inorganique obtenue,
c) éliminer l'agent porogène et au moins partiellement les ligands non échangeables qui confèrent leur caractère hydrophile aux particules.

2. Procédé selon la revendication 1 **caractérisé en ce que** les ligands non échangeables sont des silanes, dérivés stanniques ou stanneux dans lesquels l'atome de silicium ou d'étain joue le rôle de point d'ancrage du ligand sur la particule métallique.

3. Procédé selon la revendication 1 **caractérisé en ce que** les ligands non échangeables comprennent un atome de germanium qui joue le rôle de point d'ancrage du ligand sur la particule métallique.

4. Procédé selon l'une des revendications précédentes **caractérisé en ce que** la taille des murs est supérieure à 3 nm et préférentiellement comprise dans la gamme allant de 5 à 15 nm.

5. Procédé selon l'une des revendications précédentes **caractérisé en ce que** la structuration du matériau poreux est de type vermiculaire, lamellaire, hexagonal (1D ou 2D) ou cubique.

6. Procédé selon l'une des revendications précédentes **caractérisé en ce que** les ligands non échangeables comportent des groupements polaires ou polarisables, notamment choisis parmi les atomes d'halogène, tel que le chlore, les groupements amine, ammonium, phosphonate, phosphonium, hydroxyde, thiol, sulfonate, nitrate, carbonate et alcool, qui confèrent leur caractère hydrophile aux particules.

7. Procédé selon l'une des revendications précédentes **caractérisé en ce que** les ligands non échangeables sont choisis parmi le le 3-chloropropylsilane, le N-(3-trihydrogenosilylpropyl)-imidazole, le chlorobenzylsilane, le chlorodiméthylsilane, les sels de N-(3-trihydrogenosilylpropyl)alkylimidazolium ou les sels de N-(3-trihydrogenosilylpropyl)arylimidazolium, N-(benzyltrihydrogenosilyl)-imidazole, les sels de N-(benzyltrihydrogenosilyl)-alkylimidazolium ou les sels de N-(benzyltrihydrogenosilyl)-arylimidazolium, et aussi les sels de N-(benzyltrihydrogenosilyl)trialkylammonium ou le dibutyl-4,7,10-trioxaundécylstannane.

8. Procédé selon l'une des revendications précédentes **caractérisé en ce que** la charpente inorganique est constituée de silice, d'un mélange silice/oxyde de titane, d'un mélange silice/alumine.

9. Procédé selon l'une des revendications précédentes **caractérisé en ce que** les particules métalliques sont des particules de platine, ruthénium, or, nickel, cobalt, fer, argent, palladium ou rhodium.

10. Procédé selon l'une des revendications précédentes **caractérisé en ce que** la croissance de la charpente métallique est réalisée par procédé solgel.

11. Procédé selon l'une des revendications précédentes **caractérisé en ce que** le précurseur inorganique est un alcoxyde ou hydroxyde de métal ou métalloïde, de préférence un silicate, tétraalcoxysilane, tétraalcoxyde de titane ou d'aluminium.

12. Procédé selon l'une des revendications précédentes **caractérisé en ce que** la croissance de la charpente est réalisée dans un milieu aqueux ou un milieu aqueux en mélange avec au moins un co-solvant choisi parmi les alcools, de préférence les alcools linéaires tel que le butanol, les éthers tel que le THF, et le diméthylformamide.

13. Procédé selon l'une des revendications précédentes **caractérisé en ce que** la croissance de la charpente est réalisée à une température de 0°C à 100°C préférentiellement de 20°C à 65°C.

14. Procédé selon l'une des revendications précédentes **caractérisé en ce que** la croissance de la charpente est réalisée avec un rapport métal du précurseur inorganique/ agent porogène en moles de 30-300.

15. Procédé selon l'une des revendications précédentes **caractérisé en ce que** la croissance de la charpente est réalisée avec un rapport métal des particules/métal du précurseur inorganique en poids inférieur à 10%, de préférence de 0,001% à 5% et préférentiellement de 0,005 à 5 %.

16. Procédé selon l'une des revendications précédentes **caractérisé en ce que** la croissance de la charpente est réalisée dans un milieu présentant un pH de 0 à 10 et préférentiellement de 0 à 4.

17. Procédé selon l'une des revendications précédentes **caractérisé en ce que** la croissance de la charpente est réalisée en présence d'un catalyseur d'hydrolyse-polycondensation du type acide, basique ou nucléophile et préférentiellement tel que l'HCl (acide), NH₃ ou KOH, NaOH (basique) ou NaF ou TBAF (nucléophile).

18. Procédé selon l'une des revendications précédentes **caractérisé en ce que** l'agent porogène est choisi parmi :
- les templates anioniques, tel que le dodécyl sulfate de sodium ;
- les templates cationiques tels que les sels d'ammonium, les sels d'imidazolium, les sels de pyridinium ;
- les templates non-ioniques, et notamment les amines ;
- les oxydes d'alkylpolyéthylène ou d'alkylarylpolyéthylène ;
- les templates de type polysorbate ;
- les copolymères à blocs amphiphiles ;
- les polymères classiques du type PE, PP, PMMA, polystyrène.

19. Procédé selon l'une des revendications précédentes **caractérisé en ce que** l'élimination de l'agent porogène est réalisée par traitement thermique ou par dégradation en milieu aqueux sous irradiation UV, en présence d'un sel métallique.

20. Procédé selon l'une des revendications précédentes **caractérisé en ce qu'**en amont de la croissance de la charpente, on réalise une suspension colloïdale de particules de métal 0 rendues hydrophiles et stabilisées par des ligands non échangeables, additionnée de l'agent porogène, de préférence dans un mélange eau/THF.

21. Matériau poreux structuré susceptible d'être obtenu selon l'une des revendications précédentes, comportant une charpente structurée composée de murs à base d'oxyde métallique dans lesquels des particules de métal 0 sont incorporées.

22. Matériau selon la revendication 21 **caractérisé en ce qu'**il présente une surface spécifique de 20 à 1200 m²/g et préférentiellement 300 à 1100 m²/g dans le cas de charpente composée majoritairement de silice.

23. Matériau selon la revendication 21 ou 22 **caractérisé en ce que** la taille des murs est supérieure à 3 nm et, préférentiellement, comprise dans la gamme allant de 5 et 15 nm.

24. Matériau selon l'une des revendications 21 à 23 **caractérisé en ce que** la charpente inorganique est composée d'au moins un métal ou d'un oxyde métallique des groupes 3 à 11, ou d'au moins un oxyde de métalloïde d'un élément des groupes 2 et 12 à 14, ou d'un oxyde mixte de différents métaux ou métalloïdes ou d'un mélange de ces oxydes, notamment, choisis parmi les oxydes de silicium, aluminium, titane, étain, tantale ou zirconium.

25. Matériau selon l'une des revendications 21 à 24 **caractérisé en ce que** la charpente inorganique est composée de silice ou d'oxyde mixte, silice/oxyde de titane ou silice/alumine (également nommé aluminosilicates).

26. Matériau selon l'une des revendications 21 à 25 **caractérisé en ce qu'**il présente une microporosité, une mésoporosité ou une porosité mixte micro/meso.

## Patentansprüche

1. Verfahren zur Herstellung eines strukturierten porösen Materials, das ein strukturiertes anorganisches Gerüst bestehend aus Wänden auf Metalloxidbasis, in die Metall 0-Partikel eingebettet sind, umfasst, wobei das Material **gekennzeichnet ist durch** das Vorliegen wenigstens eines Beugungspeaks in einem Kleinwinkel-Röntgenpulverdiffraktogramm, das einer räumlichen Wiederholungsperiode des strukturierten Systems zugeordnet ist, die der Periodizität der Poren innerhalb des Materials entspricht,
umfassend die folgenden Schritte:
a) über eine Suspension von hydrophilen Metall 0-Partikeln verfügen, die **durch** nicht austauschbare Liganden, welche den Partikeln ihren hydrophilen Charakter verleihen, stabilisiert sind, wobei der Metallkern der Metallpartikel (ohne Liganden) im Wesentlichen sphärisch ist und wenigstens bei 50 % der Population der Partikel der Metallkern einen mittleren Durchmesser von 1 bis 10 nm aufweist, und wobei die Metallpartikel monodispers sind, das heißt, dass sie eine sehr enge Größenverteilung um einen Mittelwert aufweisen, so dass die Größe von 50 % der Partikel der mittleren Größe ± 0,5 nm entspricht,
b) das anorganische Gerüst mittels eines anorganischen Precursors um die Metall 0-Partikel herum, die **durch** die nicht austauschbaren Liganden, welche den Partikeln ihren hydrophilen Charakter verleihen, stabilisiert sind, in Gegenwart eines Porenbildners wachsen lassen, wobei die Gesamtgröße der hydrophilen Partikel, die durch die nicht austauschbaren Liganden, welche ihnen ihren hydrophilen Charakter verleihen, stabilisiert sind, kleiner als die oder gleich der Größe der Wände des erhaltenen anorganischen Gerüstes ist,
c) den Porenbildner sowie wenigstens teilweise die nicht austauschbaren Liganden, die den Partikeln ihren hydrophilen Charakter verleihen, entfernen.

2. Verfahren nach Anspruch 1, **dadurch gekennzeichnet, dass** die nicht austauschbaren Liganden Silane, Zinn(IV)- oder Zinn(II)-Derivate sind, bei denen das Silizium- oder Zinn-Atom die Rolle der Verankerungsstelle des Liganden an dem Metallpartikel übernimmt.

3. Verfahren nach Anspruch 1, **dadurch gekennzeichnet, dass** die nicht austauschbaren Liganden ein Germanium-Atom umfassen, das die Rolle der Verankerungsstelle des Liganden an dem Metallpartikel übernimmt.

4. Verfahren nach einem der vorhergehenden Ansprüche, **dadurch gekennzeichnet, dass** die Größe der Wände mehr als 3 nm beträgt und vorzugsweise im Bereich von 5 bis 15 nm liegt.

5. Verfahren nach einem der vorhergehenden Ansprüche, **dadurch gekennzeichnet, dass** die Strukturierung des porösen Materials vom Typ vermikular, lamellar, hexagonal (1 D oder 2D) oder kubisch ist.

6. Verfahren nach einem der vorhergehenden Ansprüche, **dadurch gekennzeichnet, dass** die nicht austauschbaren Liganden polare oder polarisierbare Gruppen umfassen, insbesondere ausgewählt aus den Halogen-Atomen, wie Chlor, den Amin-, Ammonium-, Phosphat-, Phosphonium-, Hydroxid-, Thiol-, Sulfonat-, Nitrat-, Carbonat- und Alkoholgruppen, die den Partikeln ihren hydrophilen Charakter verleihen.

7. Verfahren nach einem der vorhergehenden Ansprüche, **dadurch gekennzeichnet, dass** die nicht austauschbaren Liganden ausgewählt sind aus 3-Chlorpropylsilan, N-(3-Trihydrogensilylpropyl)-imidazol, Chlorbenzylsilan, Chlordimethylsilan, den N-(3-Trihydrogensilylpropyl)alkylimidazoliumsalzen oder den N-(3- Trihydrogensilylpropyl)arylimidazoliumsalzen, N-(Benzyltrihydrogensilyl)-imidazol, den N-(Benzyltrihydrogensilyl)-alkylimidazoliumsalzen oder den N-(Benzyltrihydrogensilyl)-arylimidazoliumsalzen und auch den N-(Benzyltrihydrogensilyl)trialkylammoniumsalzen oder Dibutyl-4,7,10-trioxaundecylstannan.

8. Verfahren nach einem der vorhergehenden Ansprüche, **dadurch gekennzeichnet, dass** das anorganische Gerüst aus Siliziumdioxid, einem Siliziumdioxid/Titanoxid-Gemisch, einem Siliziumdioxid/Aluminiumoxid-Gemisch besteht.

9. Verfahren nach einem der vorhergehenden Ansprüche, **dadurch gekennzeichnet, dass** die Metallpartikel Partikel aus Platin, Ruthenium, Gold, Nickel, Kobalt, Eisen, Silber, Palladium oder Rhodium sind.

10. Verfahren nach einem der vorhergehenden Ansprüche, **dadurch gekennzeichnet, dass** das Wachsen des Metallgerüstes mittels Sol-Gel-Verfahren vollzogen wird.

11. Verfahren nach einem der vorhergehenden Ansprüche, **dadurch gekennzeichnet, dass** der anorganische Precursor ein Metall- oder Metalloidalkoxid oder-hydroxid, vorzugsweise ein Silikat, Tetraalkoxysilan, Titan- oder Aluminiumtetraalkoxid ist.

12. Verfahren nach einem der vorhergehenden Ansprüche, **dadurch gekennzeichnet, dass** das Wachsen des Gerüstes in einem wässrigen Medium oder einem wässrigen Medium in Mischung mit wenigstens einem Co-Lösungsmittel, das aus Alkoholen, vorzugsweise linearen Alkoholen, wie Butanol, Ethern, wie THF, und Dimethylformamid ausgewählt ist, vollzogen wird.

13. Verfahren nach einem der vorhergehenden Ansprüche, **dadurch gekennzeichnet, dass** das Wachsen des Gerüstes bei einer Temperatur von 0 °C bis 100 °C, vorzugsweise von 20 °C bis 65 °C vollzogen wird.

14. Verfahren nach einem der vorhergehenden Ansprüche, **dadurch gekennzeichnet, dass** das Wachsen des Gerüstes mit einem Verhältnis Metall des anorganischen Precursors / Porenbildner in Mol von 30-300 vollzogen wird.

15. Verfahren nach einem der vorhergehenden Ansprüche, **dadurch gekennzeichnet, dass** das Wachsen des Gerüstes mit einem Verhältnis Metall der Partikel / Metall des anorganischen Precursors in Gewicht von weniger als 10 %, vorzugsweise von 0,001 % bis 5 % und bevorzugt von 0,005 bis 5 % vollzogen wird.

16. Verfahren nach einem der vorhergehenden Ansprüche, **dadurch gekennzeichnet, dass** das Wachsen des Gerüstes in einem Medium vollzogen wird, das einen pH-Wert von 0 bis 10 und vorzugsweise von 0 bis 4 aufweist.

17. Verfahren nach einem der vorhergehenden Ansprüche, **dadurch gekennzeichnet, dass** das Wachsen des Gerüstes in Gegenwart eines Hydrolyse-Polykondensations-Katalysators vom Typ sauer, basisch oder nukleophil und vorzugsweise wie HCl (sauer), NH₃ oder KOH, NaOH (basisch) oder NaF oder TBAF (nukleophil) vollzogen wird.

18. Verfahren nach einem der vorhergehenden Ansprüche, **dadurch gekennzeichnet, dass** der Porenbildner ausgewählt ist aus:
- den anionischen Templaten, wie Natriumdodecylsulfat;
- den kationischen Templaten, wie Ammoniumsalzen, Imidazoliumsalzen, Pyridiniumsalzen;
- den nicht-ionischen Templaten, und insbesondere den Aminen;
- den Alkylpolyethylen- oder Alkylarylpolyethylenoxiden;
- den Templaten vom Typ Polysorbat;
- den amphiphilen Blockcopolymeren;
- den herkömmlichen Polymeren vom Typ PE, PP, PMMA, Polystryol.

19. Verfahren nach einem der vorhergehenden Ansprüche, **dadurch gekennzeichnet, dass** das Entfernen des Porenbildners durch Wärmebehandlung oder durch Abbau in wässrigem Medium unter UV-Bestrahlung, in Gegenwart eines Metallsalzes vollzogen wird.

20. Verfahren nach einem der vorhergehenden Ansprüche, **dadurch gekennzeichnet, dass** vor dem Wachsen des Gerüstes eine kolloidale Suspension von durch nicht austauschbare Liganden hydrophil gemachten und stabilisierten Metall 0-Partikeln, der Porenbildner zugegeben wird, vorzugsweise in einem Wasser/THF-Gemisch hergestellt wird.

21. Strukturiertes poröses Material, das geeignet ist, nach einem der vorhergehenden Ansprüche erhalten zu werden, umfassend ein strukturiertes Gerüst bestehend aus Wänden auf Metalloxidbasis, in die Metall 0-Partikel eingebettet sind.

22. Material nach Anspruch 21, **dadurch gekennzeichnet, dass** es eine spezifische Oberfläche von 20 bis 1200 m²/g und vorzugsweise 300 bis 1100 m²/g im Fall eines hauptsächlich aus Siliziumdioxid bestehenden Gerüstes aufweist.

23. Material nach Anspruch 21 oder 22, **dadurch gekennzeichnet, dass** die Größe der Wände mehr als 3 nm beträgt und vorzugsweise im Bereich von 5 bis 15 nm liegt.

24. Material nach einem der Ansprüche 21 bis 23, **dadurch gekennzeichnet, dass** das anorganische Gerüst aus wenigstens einem Metall oder aus einem Metalloxid der Gruppen 3 bis 11 oder aus wenigstens einem Metalloidoxid eines Elements der Gruppen 2 und 12 bis 14 oder aus einem Mischoxid von unterschiedlichen Metallen oder Metalloiden oder einer Mischung aus diesen Oxiden, insbesondere ausgewählt aus den Silizium-, Aluminium-, Titan-, Zinn-, Tantal- oder Zirkonoxiden besteht.

25. Material nach einem der Ansprüche 21 bis 24, **dadurch gekennzeichnet, dass** das anorganische Gerüst aus Siliziumdioxid oder aus Mischoxid, Siliziumdioxid/Titanoxid oder Siliziumdioxid/Aluminiumoxid (auch als Aluminosilikate bezeichnet) besteht.

26. Material nach einem der Ansprüche 21 bis 25, **dadurch gekennzeichnet, dass** es eine Mikroporosität, eine Mesoporosität oder eine Mikro-/Meso-Mischporosität aufweist.

## Claims

1. A process for producing a structured porous material comprising a structured inorganic framework made up of metal-oxide-based walls in which particles of metal 0 are incorporated, said material being **characterized by** the presence of at least one diffraction peak in a small-angle X-ray powder diffractogram associated with a spatial repeat period of the structured system that corresponds to the periodicity of the pores within the material, which process, comprises the following steps:
a) formation of a suspension of hydrophilic particles of metal 0 stabilized by non-exchangeable ligands that give the particles their hydrophilic character, in which the metal core of the metal particles (excluding ligands) is essentially spherical and at least, for 50% of the particle population, the metal core has a mean diameter of 1 to 10 nm, the metal particles being monodispersed, that is to say they have a very narrow size distribution around a mean value, so that 50% of the particles have their size corresponding to the mean size ± 0.5 nm;
b) growth of the inorganic framework from an inorganic precursor around the particles of metal 0 stabilized by the non-exchangeable ligands that give the particles their hydrophilic character, in the presence of a pore-forming agent, the total size of the hydrophilic particles stabilized by the non-exchangeable ligands that give them their hydrophilic character being less than or equal to the thickness of the walls of the inorganic framework obtained; and
c) elimination of the pore-forming agent and at least partially of the non-exchangeable ligands that give the particles their hydrophilic character.

2. The process as claimed in claim 1, **characterized in that** the non-exchangeable ligands are silanes, stannic derivatives or stannous derivatives in which the silicon or tin atom acts as the point where the ligand is anchored onto the metal particle.

3. The process as claimed in claim 1, **characterized in that** the non-exchangeable ligands comprise a germanium atom that acts as the point where the ligand is anchored onto the metal particle.

4. The process as claimed in one of the preceding claims, **characterized in that** the thickness of the walls is greater than 3 nm and preferably lies in the range from 5 to 15 nm.

5. The process as claimed in one of the preceding claims, **characterized in that** the structuration of the porous material is of the vermicular, lamellar, hexagonal (1D or 2D) or cubic type.

6. The process as claimed in one of the preceding claims, **characterized in that** the non-exchangeable ligands comprise polar or polarizable groups, especially chosen from halogen atoms, such as chlorine, or amine, ammonium, phosphonate, phosphonium, hydroxide, thiol, sulfonate, nitrate, carbonate and alcohol groups, which give the particles their hydrophilic character.

7. The process as claimed in one of the preceding claims, **characterized in that** the non-exchangeable ligands are chosen from 3-chloropropylsilane, N-(3-silylpropyl)imidazole, chlorobenzylsilane, chlorodimethylsilane, N-(3-silylpropyl)alkylimidazolium salts or N-(3-silylpropyl)arylimidazolium salts, N-(benzylsilyl)imidazole, N-(benzylsilyl)alkylimidazolium salts or N-(benzylsilyl)arylimidazolium salts, and also N-(benzylsilyl)trialkylammonium salts or dibutyl-4,7,10-trioxaundecylstannane.

8. The process as claimed in one of the preceding claims, **characterized in that** the inorganic framework consists of silica, a silica/titanium oxide mixture or a silica/alumina mixture.

9. The process as claimed in one of the preceding claims, **characterized in that** the metal particles are platinum, ruthenium, gold, nickel, cobalt, iron, silver, palladium or rhodium particles.

10. The process as claimed in one of the preceding claims, **characterized in that** the metal framework is grown by a sol-gel process.

11. The process as claimed in one of the preceding claims, **characterized in that** the inorganic precursor is a metal or metalloid alkoxide or hydroxide, preferably a titanium or aluminum silicate, tetraalkoxysilane, or tetraalkoxide.

12. The process as claimed in one of the preceding claims, **characterized in that** the framework is grown in an aqueous medium or an aqueous medium mixed with at least one cosolvent chosen from alcohols, preferably linear alcohols such as butanol, ethers such as THF, and dimethylformamide.

13. The process as claimed in one of the preceding claims, **characterized in that** the framework is grown at a temperature ranging from 0°C to 100°C, preferably from 20°C to 65°C.

14. The process as claimed in one of the preceding claims, **characterized in that** the framework is grown with a molar (metal of the inorganic precursor/pore-forming agent) ratio of 30-300.

15. The process as claimed in one of the preceding claims, **characterized in that** the framework is grown with a (metal of the particles/metal of the inorganic precursor) weight ratio of less than 10%, preferably from 0.001% to 5% and preferentially from 0.005 to 5%.

16. The process as claimed in one of the preceding claims, **characterized in that** the framework is grown in a medium having a pH of 0 to 10 and preferably 0 to 4.

17. The process as claimed in one of the preceding claims, **characterized in that** the framework is grown in the presence of a hydrolysis-polycondensation catalyst of the acid, base or nucleophilic type and preferably such as HCl (acid type), NH₃, KOH, or NaOH (base type) or NaF or TBAF (nucleophilic type).

18. The process as claimed in one of the preceding claims, **characterized in that** the pore-forming agent is chosen from:
- anionic templates, such as sodium dodecyl sulfate;
- cationic templates, such as ammonium salts, imidazolium salts, pyridinium salts;
- nonionic templates, and especially amines;
- alkyl polyethylene or alkylaryl polyethylene oxides;
- polysorbate templates;
- amphiphilic block copolymers; and
- conventional polymers of the PE, PP, PMMA and polystyrene type.

19. The process as claimed in one of the preceding claims, **characterized in that** the pore-forming agent is eliminated by heat treatment or by degradation in an aqueous medium under UV irradiation, or in the presence of a metal salt.

20. The process as claimed in one of the preceding claims, **characterized in that**, prior to growth of the framework, a colloidal suspension of particles of metal 0 rendered hydrophilic and stabilized by non-exchangeable ligands is produced, to which a core-forming agent, preferably in a water/THF mixture, is added.

21. A structured porous material that can be obtained as claimed in one of the preceding claims, comprising a structured framework made up of metal-oxide-based walls in which particles of metal 0 are incorporated.

22. The material as claimed in claim 21, **characterized in that** it has a specific surface area of 20 to 1200 m²/g and preferably 300 to 1100 m²/g in the case of a framework made up predominantly of silica.

23. The material as claimed in claim 21 or 22 **characterized in that** the size of the walls is greater than 3 nm and, preferably in the range from 5 to 15 nm.

24. The material as claimed in one of claims 21 to 23, **characterized in that** inorganic framework is made up of at least one metal or an oxide of a metal of groups 3 to 11, or of at least one oxide of a metalloid of groups 2 and 12 to 14, or of a mixture of various metals or metal oxides or of a mixture of these oxides, especially those chosen form silicon, aluminum, titanium, tin, tantalum or zirconium.

25. The material as claimed in one of claims 21 to 24 **characterized in that** the inorganic framework is made up of silica or a mixed, silica/titanium oxide or silica/alumina, oxide (especially aluminosilicates).

26. The material as claimed in one of claims 21 to 25 **characterized in that** it has a microporosity, a mesoporosity or a mixed microporosity/mesoporosity.
